# EUROPEAN PATENT APPLICATION

(11) **EP 0 742 229 A2**
(43) Date of publication of application: **13.11.1996**
(21) Application number: 96106172.8
(22) Date of filing: 16.03.1991
(51) Int. Cl.: C07K 16/08, A61K 39/395

(54) **Induction of protection against viral infection**

(30) Priority: 19.03.1990 US 19900494749
(62) Divisional of application: 91906386.7
(71) Applicant: INSTITUT PASTEUR, F-75724 Paris Cédex 15 (FR)
(72) Inventor: Girard, Marc, 75015 Paris (FR); Kieny, Marie-Paule, 67100 Strasbourg (FR); Leclec, Claude, 75015 Paris (FR)
(74) Representative: Desaix, Anne, et al

(57) **Abstract**

This invention relates to monoclonal or polyclonal antibodies having neutralizing properties regarding the infection by a determined virus, obtained after administration to a host, in an amount sufficient to induce neutralizing antibodies, of a composition comprising as a combined preparation for simultaneous, separate or sequential administration:
(A) at least one envelope glycoprotein of said virus or a fragment of at least 50 aminoacids of the glycoprotein and,
(B) at least one peptide derived from the aminoacid sequence of the envelope glycoprotein, and wherein the peptide comprises at least one virus-neutralization epitope.

The invention further relates to the use of these antibodies as serotherapeutic drug.

## Description

This invention relates to a vaccination process, which involves the simultaneous or consecutive use of a priming antigen, in this case the glycoprotein from a virus, such as HIV, SIV or any lentivirus capable of inducing AIDS in its natural host, or from an HTLV-I or HTLV-II type retrovirus, and an amplifying composition comprised of synthetic oligopeptides, which are free or bound to a carrier molecule, and in which the oligopeptides correspond to the neutralization epitopes for this same glycoprotein. This invention also relates to a composition for use in the process.

An effective vaccine composition against viruses must produce rapid neutralization of the viruses in order to prevent the viruses from possibly protecting themselves in a latent provirus form within the chromosomes of resting cells or from finding refuge in the cellular or tissue compartments where they would be beyond the reach of the immune system.

From previous experiments conducted with both chimpanzees in the case of HIV and macaques in the case of SIV, it is clear that inoculation of virus envelope glycoprotein alone does not make it possible to obtain a fully protective immune response. In particular, the virus envelope glycoprotein does not produce a sufficient level of neutralizing antibodies in order to provide protection against infection.

Accordingly, there exists a need in the art for a method of inducing a sufficient level of neutralizing antibodies against virus infection in a host susceptible to the infection by the virus. In addition, there exists a need in the art for a pharmaceutical composition for use in the method.

This invention aids in fulfilling these needs in the art. An object of this invention is to reinforce the immunogenicity of at least one envelope glycoprotein of a virus by combining the glycoprotein with at least one peptide, and preferably at different times a group of peptides, derived from the sequence of the envelope glycoprotein and corresponding to virus-neutralization epitopes, i.e. corresponding to amino acid sequences involved in the production of neutralizing antibodies in the host to which they are administered.

Accordingly, this invention provides a method of enhancing the immunogenicity of an envelope glycoprotein of virus in a host and a composition for use in this method. The method comprises administering to the host at least one envelope glycopotein of the virus and at least one peptide derived from the amino acid sequence of the envelope glycoprotein. The peptide comprises at least one virus-neutralization epitope. The envelope glycoprotein and the peptide are administered in an amount sufficient to induce neutralizing antibodies in the host.

The invention provides a composition for enhancing the immunogenicity of an envelope glycoprotein of a determined virus, wherein the composition comprises as a combined preparation for simultaneous, separate or sequential use :
(A) at least one envelope glycoprotein of the virus or a fragment of at least 50 aminoacids of the glycoprotein and,
(B) at least one peptide derived from the amino acid sequence of the envelope glycoprotein, and wherein the peptide comprises at least one virus-neutralization epitope, and wherein the envelope glycoprotein and the peptide are administered in an amount sufficient to induce neutralizing antibodies in the host.

For the purpose of the invention, the word "composition" is intended to comprise combined preparation in which the components - in this case the envelope glycoprotein and the peptide or peptides derived from the envelope glycoprotein - can be presented in a mixture or can be presented side-by-side and therefore be applied simultaneously, separately or at intervals, to the host.

For instance the peptide(s) present in the composition can be maintained separated from other components in order to be administered sequentially to booster the immonogenic reaction which is primed with the envelope glycoprotein.

In a preferred embodiment, the invention provides a composition which comprises the above envelope glycoprotein and peptide providing the envelope glycoprotein is present in an amount sufficient for priming the induction of neutralizing antibodies in a host to which it is administered; and the at least one peptide is in an amount sufficient to enhance the induction of persistent neutralizing antibodies in the host to which it is administered.

Accordingly the invention concerns the use of at least one of the above described peptides for enhancing the immunogenicity of tan envelope glycoprotein of a virus, when this glycoprotein is administered to a host to induce neutralizing antibodies.

The composition of the invention can be used for the preparation of an immunotherapeutic drugs. In this case the composition is administered to seropositive people in order to increase the level of neutralizing antibodies and accordingly to enable a control of the virus.

Methods described by J. Salk in "4• Colloque des Cent Gardes - Retroviruses of human AIDS and related animal diseases - Ed. M. Girard, L. Valette - Fondation Mérieux - 1990 p.273-278" and in "Nature 1989, vol. 327 p.473-476".

This invention also provides a composition for vaccinating a host against infection by a virus. The composition comprises at least one envelope glycoprotein of the virus in an amount sufficient for priming vaccination in a host to which the envelope glycoprotein is administered. The composition also contains at least one peptide derived from the amino acid sequence of the envelope glycoprotein. The peptide comprises at least one virus-neutralization epitope of the glycoprotein. The composition contains the peptide in an amount sufficient to enhance the induction of persistent neutralizing antibodies in the host.

The description of the invention in connection with the use as vaccine of the defined composition can also be applied to the use as immunotherapeutic drug of this composition, provided that the described means enables the enhancement of the production of neutralizing antibodies.

Peptides and envelope glycoproteins can be combined under conditions allowing them to interact by non-covalent physical combination or by covalent chemical bonding. Alternatively, and in a preferred embodiment of the invention, a priming vaccination (priming) is achieved by injections of envelope glycoprotein, with protective immunity being subsequently enhanced by the injection of immunogenic peptides corresponding to the neutralization epitopes.

Two of three immunized chimpanzees were successfully protected against virus infection and virus was suppressed in a third animal for a long period using the compositions and methods of this invention. These results demonstrate that this invention makes it possible to elicit protection against HIV-1 through immunization.

### Brief Description of the Drawings

This invention will be more fully described by reference to the following Figures in which:
Fig. 1 depicts anti-HIV antibody level measured by ELISA (Genetic Systems Kit) in chimpanzee FUNFACE (C-339) and a control (C-519). The results are shown as serum ELISA titre (1:dilution giving positive response) versus time.
Time zero in the Figure corresponds to the day of the first booster with inactivated HIV The animal was challenged at 70 weeks (arrow).
Fig. 2 depicts neutralizing antibody level in chimpanzees FUNFACE (dark circles) and ROBERT (open circles) in response to the injection of a KLH-BRU peptide conjugate (arrows). The animals were inoculated at 0, 3, and 19 weeks (arrows) and challenged at 24 weeks.
Fig. 3 depicts anti-HIV antibody levels measured by ELISA in chimpanzee ROBERT (C-433). The results are shown as serum ELISA titre (1:dilution giving positive response) versus time. Time zero corresponds to the day of the first antigen injection (gp160env, p27nef, p23vif, and p18gag). The animal was challenged at 84 weeks (arrow).
Fig. 4 depicts neutralization of HIV-1 BRU as a function of the serum dilution in chimpanzees JOJOTOO (499), IRA (151), and HENRY II (531) at time t0( 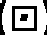 ) and at 2 weeks (□) and 5 weeks (◆) after a third inoculation of free peptides.
Fig. 5 depicts neutralization of HIV-1 BRU (dotted curves) and HIV-1 ARV-2 (solid curves) as a function of the serum dilution in chimpanzee JOJOTOO (C-499) at time t0 ( 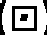 ) and after the third inoculation of free peptides (◆).
Fig. 6 shows total HIV-1-specific antibody titers for chimpanzees C-339 (A), C-433 (B), and C-499 (C). At the indicated times, chimpanzees were inoculated with various immunogens (see Table 1) or challenged with HIV-1. Titers are defined as the reciprocal of the highest dilution of serum that was positive using an HIV-1 EIA kit (Genetic Systems).
Fig. 7 depicts neutralizing antibody titers in serum from C-339, C-433 and C-499 during immunization with HIV-1 antigens. Titers are the reciprocal of the highest dilution of serum that gave 90% reduction in number of syncytia formed by CEM-SS cells (Nara, P.L., Hatch, W.C., Dunlop, N.M., Robey, W.G., Arthur, L.O., Gonda, M.A. & Fischinger, P.J. (1981) AIDS Res. Human Retroviruses 3, 283-302.) when compared to that obtained with control serum from a naive chimpanzee.
Fig. 8 shows PCR analysis of DNA from PBMC and lymph node tissue obtained 6 months after challenge of chimpanzees C-339 and C-433 with HIV-1.
   (A) Ethidium bromide-stained gel of amplified HIV sequences following two rounds of PCR with nested sets of primers. The size of the HIV-specific amplified fragment is 141 base pairs.
      Lane 1, 0.5 µg of OX174 DNA cleaved with *Haelll* as molecular weight markers.
      Lanes 2-7, positive controls for sensitivity, each containing tenfold fewer molecules of pHXB2 cleaved with *Xbal* than the previous sample, starting with 3000 molecules in lane 2. Each sample was amplified in the presence of 1 µg DNA (the amount of DNA in 1.5 x 10⁵ cells) from an uninfected control chimpanzee, C-519. One negative control sample (lane 14) was identified and used as a source of uninfected chimpanzee cellular DNA; all other samples were tested blindly. C-487 was an HIV-1 infected chimpanzee, used as a positive control.
      Lanes 8-11, DNA from PBMC of C-339, C-487, C-433 and C-519, respectively.
      Lanes 12-15, DNA from lymph node tissue of C-487, C-433, C-519 and C-339, respectively.
   (B) Ethidium bromide-stained gel of an amplified portion of the beta-globin gene (Scharf, S.J., Horn, G.T. & Erlich, H.A. (1986) Science 233, 1076-1078), as an internal control. (C) Oligonucleotide hybridization of PCR-amplified sequences. PCR reaction products shown in (A) were denatured and annealed with [³²P]-labeled primer SK102, which anneals entirely within the amplified sequence; the products were examined following polyacrylamide gel electrophoresis and authoradiography according to Kwok and Kellogg (Kwok, S. & Kellogg, D.E. (1990) in PCR Protocols: A Guide to Methods and Applications: eds. Innis, M.A., Gelfand, D.H., Sninsky, J.J. & White T.J. (Academic Press, Inc., San Diego, CA) pp. 337-347).

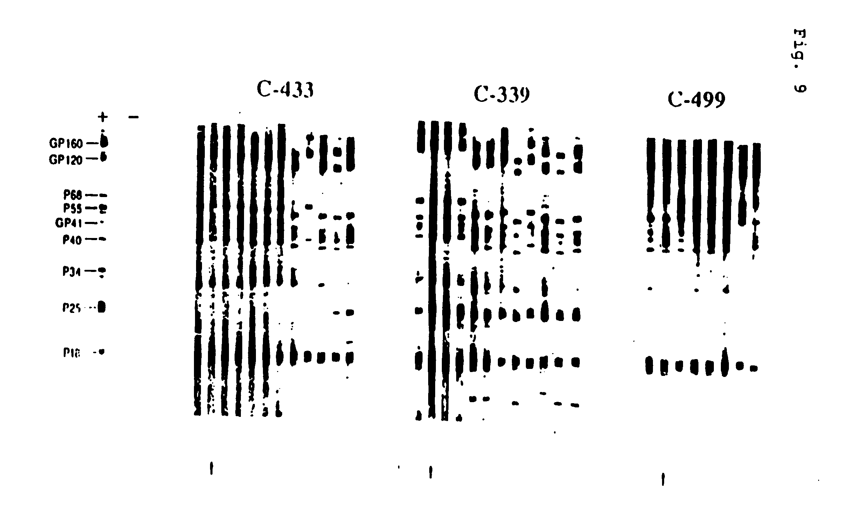

Fig. 9 depicts immunoblot analysis of antibodies to specific HIV-1 proteins following immunization and challenge of chimpanzees C-433, C-339 and C-499. Serum samples were diluted 1:200 and tested with a commercial kit (Diagnostics Pasteur). For the samples shown, sera were collected one month prior to challenge (marked by arrow) and then at 4 week intervals. Molecular weights of HIV-1 proteins are shown for positive control serum.

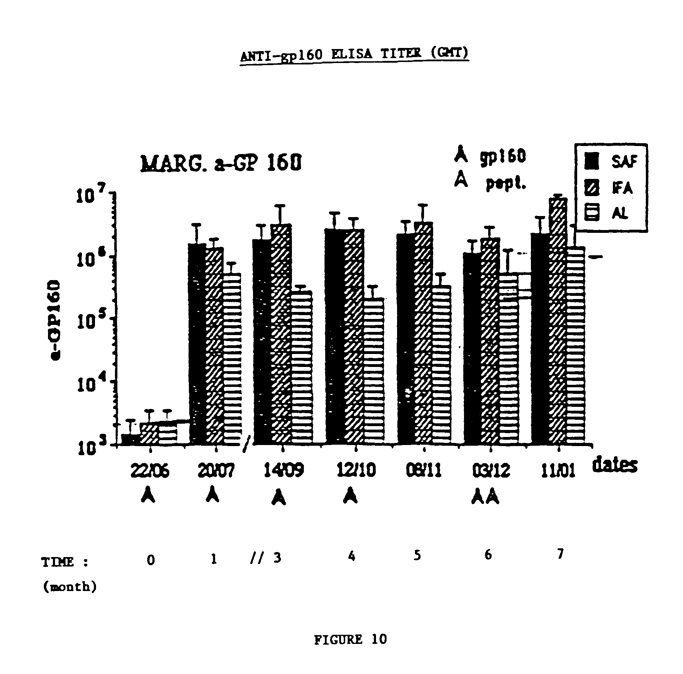

Fig. 10 shows anti-gp160 ELISA titers in Rhesus monkeys treated according to the invention.

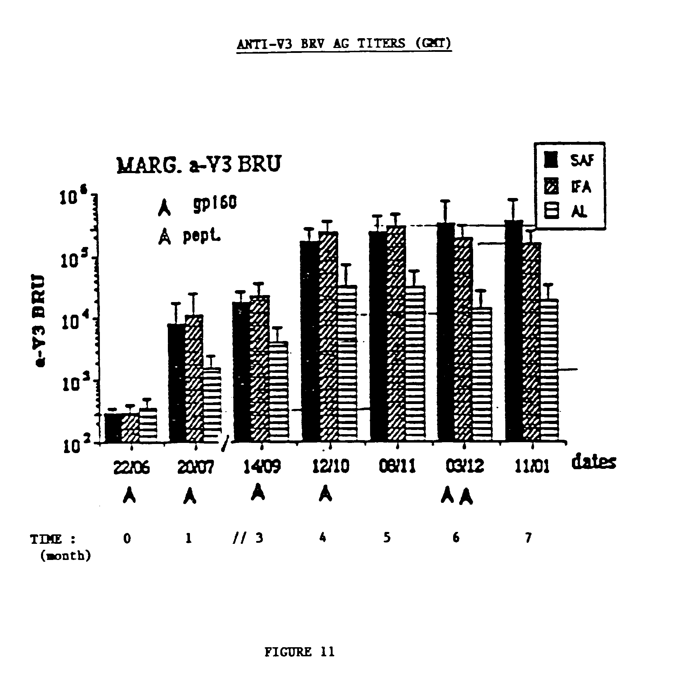

Fig. 11 shows anti-V3 BRU antibody titers in Rhesus monkeys treated according to the invention.

### Best Mode for Carrying Out the Invention

Previous attempts to protect chimpanzees against HIV infection by vaccination have failed, despite the use of several different types of vaccines: synthetic peptides, live recombinant vaccinia virus (VV) expressing HIV antigens, native or recombinant gp120 or gp160 envelope antigens, and inactivated whole virus. The failure to protect a chimpanzee against an infectious HIV challenge by prior vaccination with recombinant VV followed by formalin- and betapropiolactone-inactivated whole HIV was previously reported.

This failure led to two considerations on which the present approach is based:
1 Protection against infection with cell-free HIV probably requires high levels of neutralizing antibodies (Ab). Should the virus escape eradication by neutralizing Ab or antibody dependent cellular cytoxicity (ADCC), the virus could easily remain sheltered from the immune system, either as an integrated provirus and/or by infection of cells in the bone marrow or central nervous system. Replication of the virus, even if limited, could lead to the early emergence of neutralization escape mutants. Therefore, rapid neutralization of the challenge virus may be a key to successful vaccination.
2 Up to 1990, induction of neutralizing Ab by all the vaccines tested in chimpanzees has been at best mediocre. This may explain their failure to protect the animals against infection. To be efficacious, a vaccine, therefore, should induce higher neutralizing Ab titers than those obtained so far.

It was, therefore, sought to elicit the highest possible neutralizing Ab titers in chimpanzees through successive immunization protocols using a variety of immunogens.

One chimpanzee, C-339, was immunized initially with four injections (at 0, 1, 2 and 6 months) of 250 µg of formalin and betapropiolactone-inactivated whole HIV mixed with SAF-1 using a concentration of 1 mg threonyl MDP. The animal developed high HIV ELISA titers (1:200,000, using the ELAVIA kit from Diagnostic Pasteur with a cut off of 0.1) and showed strong reactivity by Western blot to gp160, gp120, and gp41 env, and to p55, p40, p25, and p18gag. Its neutralizing Ab titers reached 1:400 and 1:64, respectively, using two different neutralization assays; the first assay scored for 50% inhibition of immunofluorescent foci formation on MT4 cells, and the second one for 90% inhibition of syncytia formation on CEM-SS cells. Using a more stringent assay (100% inhibition of reverse transcriptase production in fresh human PBL), the maximum titer of neutralizing Ab was 1:160, obtained immediately after the booster injection. These titers, however, did not persist, but quickly declined to lower levels.

In an attempt to increase the neutralizing Ab titers of chimpanzee C-339, the animal was boosted repeatedly with recombinant soluble gp160env purified from the supernatant of BHK-21 cell cultures infected with VV-1163, a VV-env recombinant expressing a gp160 molecule containing a deletion of the transmembrane domain and a modification by site-directed mutagenesis of the gp120/gp41 cleavage site to prevent cleavage. Vaccinia virus VV-1163 can be made using the procedures described by Kieny et al., Protein Engineering 2:219-226 (1988). The antigen was purified by sequential lectin and cation-exchange chromatography, then was injected I.D. at multiple sites of the chest (125-150 µg per injection) with a human dose of BCG. This was followed by 3 successive I.M. injections of the antigen formulated with SAF. ELISA and neutralizing Ab titers were followed on routinely; however, both remained unchanged during and after this course of immunizations.

Failure of the gp160env to enhance antibody responses was not due to lack of immunogenicity, as shown by immunizing in parallel a naive chimpanzee, C-519, which previously had not been exposed to HIV antigens. Using the same immunization protocol as for C-339, C-519 readily developed a strong anti-gp160 Ab response, and its ELISA titer reached 200,000 after two injections. Therefore, failure of C-339 to respond to the injections of gp160env was not due to lack of potency of the immunogen, but most likely to some unidentified, immunological block in the animal. It was reasoned that such an impairment might be by-passed by injecting the animal with only those epitopes of the gp120 molecule that were required for induction of neutralizing Ab.

It has been shown that HIV neutralizing Ab are primarily directed against the type-specific, hypervariable loop from the V3 region of gp120. Therefore, using bis-diazobenzidine, a 25-mer oligopeptide with the sequence of that loop 4(YNTRKSIRIQRGPGRAFVTIGKIGN) from the HIV-I BRU (IIIb) strain was cross-linked to KLH. C-339 was injected with the peptide-carrier conjugate in the presence of SAF (300 µg of peptide) at 0, 3, and 19 weeks. No increase in ELISA titer was observed, but sustained neutralizing Ab titers were obtained following the second injection. The animal was challenged on week 26 (see below), together with another chimpanzee, ROBERT, C-433, that had undergone a parallel, albeit distinct, course of immunization.

Chimpanzee C-433 had been primed with VV-1139, a VV recombinant expressing the same uncleaved version of gp160env as VV-1163, but containing the transmembrane domain. Vaccinia virus VV-1139 can be made using the procedures described by Kieny et al., Protein Engineering 2:219-226 (1988). Scarification was done with 2 x 10⁸ PFU of the VV recombinant and was repeated at 4 and 22 weeks. The animal was then immunized with 125-150 µg each of recombinant soluble gp160, purified as described above, and recombinant p18gag, p27nef and p23vif (purified from *E*. *coli*) mixed with SAF. Injections were at 0, 1, 2, and 6 months, and resulted in an ELISA Ab titer of 1:400,000. Again, however, neutralizing Ab titers remained low (1:400 and 1:128, by the immunofluorescent focus and syncytia-forming assays, respectively). C-433, therefore, was injected with the same V3 peptide-KLH conjugate, according to the same immunization protocol, as C-339. The neutralizing Ab titer of C-433 was immediately boosted several fold and the animal was challenged in parallel with C-339.

The two chimpanzees were challenged using a titrated virus stock (III B stock, lot No. 40) from the National Cancer Institute (a kind gift of Larry Arthur, NCI, Frederick, MD). The stock, which contained 10⁴ TCID50/ml, was diluted 1:100, and 1 ml of the dilution was injected I.V. into both of the immunized animals. To prevent unnecessary use of an animal, and in view of the fact that the virus stock had been titrated twice in chimpanzees and its infectivity for chimpanzees had been assessed regularly, no control naive chimp was used in this experiment. The chimp ID50 of this virus stock was equivalent to 4 TCID50, and in two experiments, injection of chimpanzees with 40 TCID50 resulted in the appearance of detectable virus in PBL as early as 2 weeks after injection and was followed by seroconversion at 4 weeks.

By contrast, the challenge of chimpanzees C-339 and C-433 with 100 TCID50 was not followed by detectable increases in antibody titers during the 24 weeks that have elapsed since time of challenge. In addition, C-433 has not developed anti-p25gag Ab, C-339 has not developed anti-p27nef Ab, nor have the 2 animals developed anti-p66pol Ab.

PCR tests done at 6 weeks, 12 weeks, and 24 weeks after challenge on PBL from both chimpanzees were negative, whereas the insufficiently immunized chimpanzee (C-487) that was challenged and became infected a year ago, was positive by PCR. Finally, virus has not been recovered by cocultivation of PBL from either C-339 or C-433 with human PBL, as judged by absence of RT activity after 6 weeks of culture.

It is understood that the expression "neutralization epitopes" is taken to mean, in the case of HIV-1, the major virus-neutralization epitope, such as described, among others, by Putney et al. in 1986 (Science 234:1392-1395) and by Rusche et al. in 1988 (Proc. Natl. Acad. Sci. USA 85:3198-3202), for which the sequence corresponds approximately to amino acids 296 to 331 of the HIV-1 envelope glycoprotein as described in the work of Myers et al. (Human Retroviruses and AIDS 1989, Los Alamos, Natl. Lab). Also covered by the invention are peptides corresponding to equivalent regions of different variants of HIV-1, or another retrovirus, HIV-2, HTLV-I, or HTLV-II in humans, FIV, FeLV, or another lentivirus in animals, and which correspond to the neutralization epitopes of the virus under consideration.

Also included in the scope of the invention are peptides corresponding to those known as minor neutralization epitopes, characterized by the fact that they belong to conserved regions of the envelope glycoprotein, and that they induce antibodies capable of neutralizing, at relatively low titers, several different isolates of the virus under consideration, for example several different isolates of HIV-1, or even different isolates of HIV-1, and also of HIV-2. An example of a minor epitope can be found in the work of Chanh et al. in 1986 (The EMBO Journal, 5:3065-3071) and in that of Evans et al. in 1989 (Nature 339:385-388), or Almond et al. in "Retroviruses of human AIDS and related animal disease," M. Girard and L. Valette, Foundation Marcel Merieux, Lyon, 1990, in press).

Immunogenic peptides of major and minor neutralization epitopes are preferably mixed with each other to ensure the greatest possible protection. They can be administered in the free state, not coupled to a carrier molecule. They can also be combined with a sequence of amino acids having one or preferably several T-epitopes from one or several structural or non-structural proteins of the same retrovirus or a retrovirus immunologically cross-reactive with the former, particularly such as described in French patent application of Girard-Gluckman-Bahraoui, No. 89.11044 of August 18, 1989.

In one particularly preferred embodiment of the invention, immunogenic peptides corresponding to neutralization epitopes are chemically coupled to sequences of amino acids corresponding to T-epitopes. In another case, the peptides are coupled to a carrier molecule which bears the desired T-epitopes, by allowing them to react, for example, with a bifunctional reagent or any other coupling agent desired.

As a carrier molecule, any protein coded for by the viral genome can be used (in the case of HIV, the proteins produced by tat, rev, vif, pol, vpr, vpx, vpu, gag, env, or nef genes), or other (protein-type) molecules, such as HBs antigen, HBc antigen, tetanus toxoid, hemocyanin, human albumin, or polypeptides (for example polylysine) or appropriate lipopeptides.

In a particular embodiment of the invention in which the envelope glycoprotein molecules and major and minor neutralizing peptides (either free or bound to carrier molecules) are combined in the same vaccine preparation, the priming effect of the envelope glycoproteins appears after the first one or few injections of vaccine, and the amplification effect due to peptides immediately afterward.

Thus, an object of the invention is to use a first antigen, in this case the several envelope glycoproteins of each of the retrovirus serotypes under consideration, which has the effect of priming the response of the immune system; and a second antigen, in this case the synthetic peptides corresponding to major and also possibly minor neutralization epitopes of the different serotypes of the virus under consideration, for vaccination (preferably consecutively, but in a mixture, if necessary) with the purpose of amplifying and consolidating the initial response, particularly through induction of long-lasting, high-titer neutralizing antibodies. This invention makes it possible to induce immunity that persists as long as about six months and even as long as one year or more.

The glycoproteins used to prime the response of the immune system are preferably whole molecules as obtained before possible cleavage. Thus, in the case of HIV-1, gp160 is preferable to gp120, and the same is true for other retroviruses. This allows anti-gp41 antibodies in particular to be induced, which is a favorable sign in virus carriers (Klasse et al., Proc. Natl. Acad. Sci. USA, 85:5225-5229).

The peptides constituting the "amplifier" can be free or physically bound (especially by hydrophobic bonding) or chemically bound (especially by covalent bonding) to carrier molecules. They can also be associated with other peptides corresponding to T-epitopes, or even to peptides, lipopeptides, glycopeptides, aliphatic chains, fatty acids, or any combination of these capable of stimulating the immune system and/or specifically targeting the "amplifier" peptides to antigen-presenting cells.

From this point of view, a particularly advantageous presentation of peptides corresponding to HIV neutralization epitopes is to bind them, preferably by covalent chemical bonding, to an aliphatic sequence, particularly as described in 1989 by Deres et al. (Nature 342:561-564). The amplifying peptides presented in this way can induce not only a B-cell response, but also a CTL CD8⁺ response, restricted HLA Class I, as described by Takanashi et al. in 1988 (Proc. Natl. Acad. Sci. USA 85:3105-3109).

When the virus has a high degree of antigenic variability, as in the case of HIV-1 and HIV-2, it is necessary to use as priming antigen not just one, but several envelope glycoproteins with different sequences, each sequence corresponding to an isolate or group of isolates of the virus under consideration, so as to obtain as many priming phenomena as desired, since each is specific for a single isolate or group of isolates. In this case, it is understood that the amplifying peptides are composed of the mixture of neutralization peptides of each of the isolates under consideration, as indicated below.

A preparation of HIV-1 amplifier peptides according to the invention is characterized by the fact that it contains at least one of the sequences or one part of the sequences described below in one letter amino acid code:
C-TRPNNNTRKR IRIQRGPGRA FVTIGK-IGN M-RQAH-C
C-TRPNNNTRKS IRIQRGPGRA FVTIGK-IGN M-RQAH-C
C-TRPNNNTRKK IRIQRGPGRA FVTIGK-IGN M-RQAH-C
C-TRPNNNTRGS IRIQRGPGRA FVTIGK-IGN M-RQAH-C
C-TRPNNNTRKS IYI--GPGRA FHTTGRIIGD -IRKAH-C
C-TRPYNNVRRS LSI--GPGRA FRTRE-IIGI -IRQAH-C
C-TRPGNNTRRG IHF--GPGQA LYTTGIV-GD -IRRAY-C
C-ARPYQNTRQR TPI--GLGQS LYTTRSR-SI -IGQAH-C
C-TRPNNNTRKS ITK--GPGRV IYATGQIIGD -IRKAH-C
C-TRPNNNTRKR ITM--GPGRV YYTTGQIIGD -IRRAH-C
C-TRPGSDKRQS TPI--GLGQA LYTTRGRTKI -IGQAH-C
C-TRPGSDKKIR QSIRIGPGKV FYAKGG---I -TGQAH-C
C-TRPNNNTKKG IAI--GPGRT LYAREKIIGD -IRQAH-C
C-TRPNNHTRKR VTL--GPGRV WYTTGEILGN -IRQAH-C
C-TRPGNNTRRG SHF--GPGQA LYTTGIVGDI -RRAY-C
C-TRPDNKITSRQ-TPI-GLGQA LYTTRIKGDI -RQAY-C
C-TRPNNNVRRR-HIHI-GPGRA FYTGEIRNI -RQAH-C
C-TRPYKNTRQS-TPI--GLGQA LYTTRTKSI -GQAH-C
C-TRPNNNTTRS-IHI--GPGRA FYATGDIIGTIRQAH-C
C-TRPNYNKRKR-IHI--GPGRA FYTTKNIIGDIRQAH-C

The production of the amplifying molecules of the invention by using a sequence containing at least one neutralization epitope and particularly one of those from the list above and one carrier sequence having at least one T-epitope, may be achieved by binding these sequences or by physical combination in the same composition.

To be fully effective, priming and amplifying antigens must be enhanced, for example and preferably by lipid adjuvants, such as derivatives of muramyl dipeptide in lipid emulsions, or incomplete Freund's adjuvant.

The priming and amplifying antigens are preferably administered intramuscularly to a host, such as a primate, and especially a human. Following are typical immunization schedules that can be employed for gp160 and peptides of HIV

| gp160 (months) | Peptides (months) |
|---|---|
| 0, 1, (2), 6 | 12, 13 |
| 0, 1, 2, 12 | 13, 14 |
| 0, 1, 2, 12 | 1, 2, (12) |

It will be understood that these immunization schedules are merely representative and that the schedules can be varied to obtain the optimum response in the host. Similarly, the amounts of the priming and amplifying antigens can be varied. For example, about 150 µg of gp160 in Syntex SAF-1 adjuvant can be administered as indicated, followed by administration of the peptides in amounts of typically 100 µg of each peptide.

Finally, the relative proportions of the peptides involved can vary according to the desired final proportions of each peptide in the final preparation. In particular, these proportions will be adjusted as a function of the immunogenicity of each peptide and the number of functional groups carried by each one, which are capable of entering into the conjugation reaction with complementary functional groups, at least when these peptides are coupled to a carrier molecule.

In a particular application of the, invention, the injection of amplifying peptides is replaced by the administration of particles, virus, or bacteria, which are recombinants expressing the neutralization epitope of the virus under consideration on their surface and/or during their multiplication and in this way are capable of inducing neutralizing antibodies against said retrovirus: HBc antigen particles; HBs antigen particles; bacteria expressing the neutralization epitope in surface or cytoplasmic proteins, such as, for example, the lamB receptor; picorna virus chimeras, such as, for example, poliovirus-HIV chimeras; poxvirus recombinants; adenovirus recombinants or adenovirus chimeras, etc. Depending on the live vector selected for the presentation of the neutralization epitope, this administration can be carried out in the form of live vaccine administered orally (for example, chimeras constructed from Sabin poliovirus strains or from human adenoviruses, or from attenuated strains of Salmonella, Shigella, or other enterobacteria, or from any organism, virus, yeast, bacteria, capable of inducing an immune response after oral administration) or in the form of live vaccine administered by the parenteral route (for example, recombinant poxvirus) or even in the form of inactivated vaccine by the parenteral route (for example, chimeras constructed from the Mahoney strain of poliovirus, or inert particles of HBsAg or HBcAg).

In another particular embodiment of the invention the antigen (envelope glycoprotein) which is injected for the priming of the vaccination, i.e. the envelope glycoprotein of the virus, is presented under the form of particles such as ISCOMs (Immuno Stimulating COMplex, consisting in an association of an antigenic protein with a glycoside Quil A) or liposomes.

The priming antigen and/or the peptide can be also associated with live recombinant microorganisms such as viruses or bacteria (for instance the poxvirus or BCG: Bacille de Calmette Gérin) or any live vaccine modified to express the envelope glycoprotein or the peptide derived therefrom.

The envelope glycoprotein and/or the peptide derived therefrom can also be presented by inactivated particles, for instance viral particles such as the HIV virus or a part of this virus, or particles without genome.

Such particles without genome have been described to produce vaccine by Haffar O. et al - Journal of Virology-June 1990 p.2653-2659.

These particles can ba called HIV-like particles in the case of HIV virus: For the purpose of the invention they do not contain the complete HIV genome but they enable the exposition at their surface, of the virus components of the composition of the invention.

In another embodiment of the invention, the envelope glycoprotein antigen is combined in a mixture with other antigens. For instance when the priming antigen is the HIV envelope glycoprotein, one or several antigens such as, gag, nef, vif, pol, GPG or GLG antigens can be combined with it, as they can be combined with the peptides of the composition.

The invention also comprises the compositions above described, wherein the env glycoprotein is replaced by or associated with a fragment thereof. This fragment has advantageously more than 50 aminoacids and is characterized in that he as the immunogenic properties of the glycoprotein in the context of the invention.

The invention also concerns monoclonal or polyclonal antibodies which recognized the glycoprotein and/or peptides of the composition. This antibodies can be associated in a mixture and used for instance for serotherapeutic purpose.

### EXAMPLE 1: Immunization of a chimpanzee with HIV-1 BRU and the glycoprotein of this isolate; amplification of the response with a BRU env oligopeptide coupled to KLH.

Chimpanzee 339 (FUNFACE) was first immunized with three injections at one month intervals of 250 µg of purified HIV-1 BRU virus, inactivated by treatment with 0.025 percent formalin for 48 hours at 30°C and 0.025 percent betapropiolactone for 30 minutes at 37°C, combined with Syntex adjuvant containing 1 mg/ml threonyl-MDP in an emulsion of 5 percent squalane and 2.5 percent pluronic polymer. These injections were followed by a first booster at 7 months and a second booster one year later.

The animal then received five injections of BRU virus envelope glycoprotein (gp160) purified from supernatant of BHK-21 cell cultures infected with a vaccinia virus recombinant (strain VVenv 1163) having a genome for which genetic recombination techniques were used to insert the sequences of HIV-1 BRU coding for gp160env modified through oligonucleotide site-directed mutagenesis to eliminate the sequences involved in gp120/gp41 cleavage and from which the transmembrane hydrophobic zone was deleted, as described in Kieny et al. in 1988 (Prot. Engineering 2:219-226). The purified protein was used in an amount of 125-150 µg per intramuscular injection in the presence of Syntex adjuvant. To prepare the glycoprotein, the culture medium of BHK cells infected with VV-1163 was concentrated by precipitation with ammonium sulfate, then with trichloracetic acid, and the glycoprotein was then purified by three successive runs of affinity chromatography over lentil lectin, ion exchange over cation-exchange resin, and high-performance liquid chromatography (HPLC). The recombinant gp160 obtained in this way is 95 percent pure. It is recognized by monoclonal antibodies specific of the gp160 of HIV-1 and particularly by neutralizing antibodies 110-4 specific for the major neutralization epitope of the BRU isolate. Moreover, it shows a strong affinity for the CD4 receptor of T4 lymphocytes.

The level of antibodies induced in response to injections of inactivated virus (ELISA determination: 1/200,000 with the Diagnostics Pasteur ELAVIA kit; neutralizing titer: 1/400 by measurement of 50% inhibition of the formation of immunofluorescence foci; 1/64 by measurement of 90% inhibition of syncytia formation in CEM-SS cells), was not changed appreciably by the injection of gp160.

The animal was given 300 µg of preparation of synthetic peptide having the sequence Y N T R K S I R I Q R G P G R A F V T I G K I G N corresponding to the neutralization epitope of the BRU isolate, the tyrosine residue (Y) being coupled to hemocyanine (KLH) with bis(diazobenzidine) and combined with Syntex adjuvant. The injection was repeated once three weeks later, then a second time at 19 weeks.

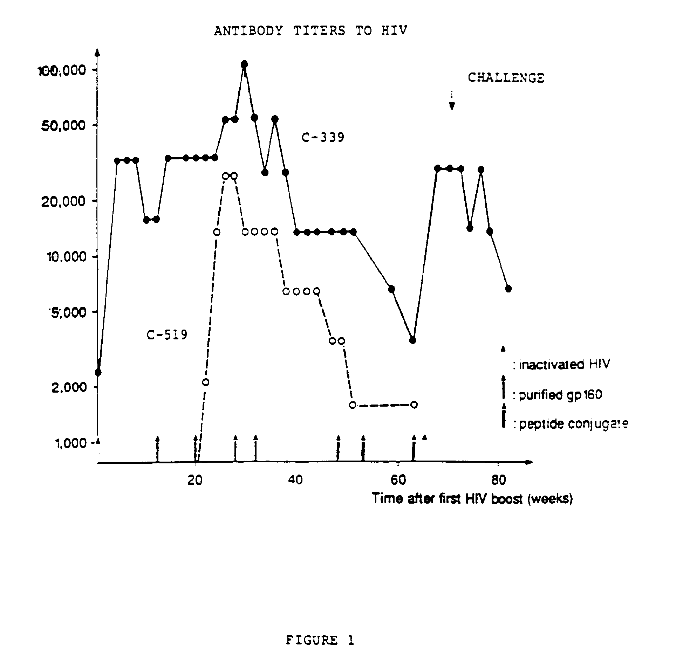

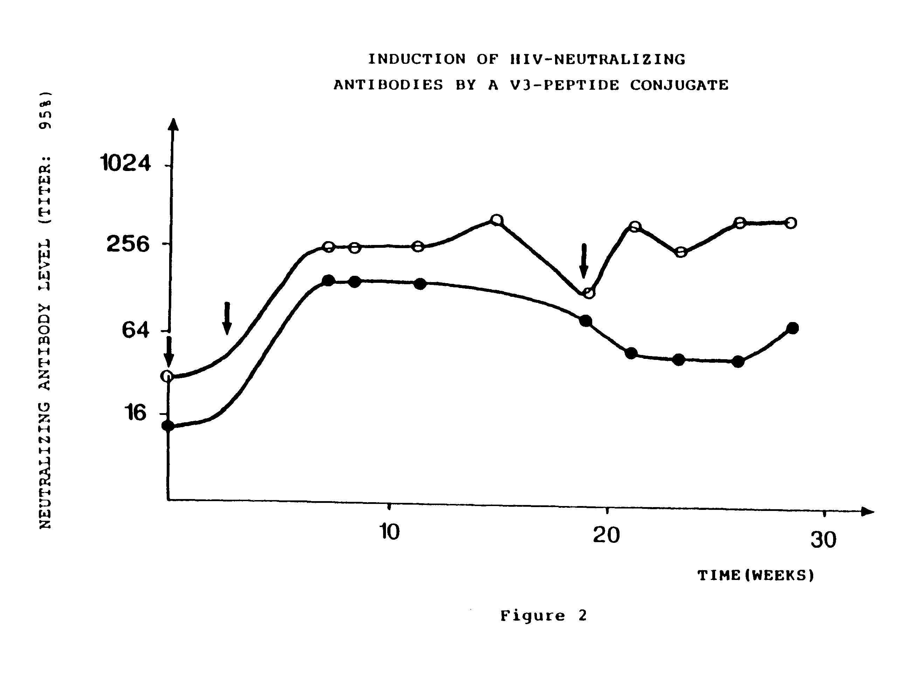

These injections did not result in any increase in antibody titers measured by ELISA (Figure 1), but they did result in a marked increase in neutralizing antibodies, as can be seen in Table 1 and Figure 2, as measured by three different antibody titration methods.

**Table 1**

| Induction of neutralizing antibodies in the chimpanzee FUNFACE (C-339) | | | |
|---|---|---|---|
| Date after 1st injection (weeks) | Level of neutralizing antibodies measured by method | | |
| | A | B | C |
| 0 | 0 | 32 | 100 |
| 3 | 100 | | 150 |
| 8 | 1600 | 128-256 | 800 |
| A: 90% inhibition of syncytia in MT4 cells | | | |
| B: 90% inhibition of syncytia in CEM-SS cells | | | |
| C: 75% inhibition of immunofluorescence in H9 cells | | | |

FUNFACE was then challenged at 26 weeks, by administering an intravenous injection of 1 ml of a 1:100 dilution, or 100 TCID50 of a HIV-1 stock titrating 10⁴TCID50/ml, kindly provided by Larry Arthur (NCI, Frederick). This stock 040 was titered on two occasions in the chimpanzee, which allowed Arthur et al. to determine that its ID50 for the chimpanzees wan 4 TCID50. The injection of 40 TCID50 of thin stock in unimmunized chimpanzees resulted in the appearance of detectable virus in the lymphocytes of the animal starting two weeks after injection and was followed by anti-HIV seroconversion within four weeks, as observed in the two samples, and as published by Arthur et al. in 1989 (J. Virol.).

The chimpanzee FUNFACE demonstrated apparently total protection against infection with 100 TCID50 of the stock 040 virus, because at up to six months after the challenge injection, no virus was detected in his lymphocytes (as measured either by gene amplification with pol and gag probes, or by coculture with human lymphocytes and assay of reverse transcriptase in 100,000 x g pellets obtained from culture supernatants) and at six months, there was no anti-HIV anamnestic response as measured by ELISA or by Western blot (Table 2) and no anti-nef antibody detectable by Western blot.

**Table 2**

| Fate of anti-gp160 and anti-major BRU neutralization epitope antibodies after challenge injection of FUNFACE | | | | | |
|---|---|---|---|---|---|
| ELISA titer on date indicated | | | | | |
| Antigen | day of challenge | +1 month | +2 months | +3 months | +4 months |
| gp160 | 179,000 | 127,000 | 89,000 | 44,000 | 18,000 |
| BRU peptide | 6,000 | 3,000 | 2,500 | 1,000 | 1,000 |

### EXAMPLE 2: Immunization of a chimpanzee with recombinant antigens env, gag, nef, and vif of HIV-1; amplification of the response by a BRU env oligopeptide coupled to KLH.

Chimpanzee 433 (ROBERT) was first primed with three consecutive scarifications of 2 x 10⁸ PFU of a recombinant vaccinia virus (VVenv 1139) expressing the gp160env of HIV-1 BRU, then by the intravenous administration of his own lymphocytes which previously had been infected in vitro by the recombinant virus VVenv 1139 and fixed in formaldehyde. The animal then received three consecutive intramuscular injections at one month intervals, then three boosters at 33, 38, and 40 weeks and a last booster at 66 weeks consisting of a mixture of 125-150 µg of each of the following antigens combined with Syntex adjuvant: gp160env, purified as described in Example 1 above, and the proteins p18gag, p27nef, and p23vif expressed in E. coli and purified as described in French patent application No. 89.11044 of August 18, 1989. Finally, ROBERT received the same BRU peptide coupled to KLH and combined with Syntex adjuvant on the same inoculation schedule as FUNFACE did in the previous example.

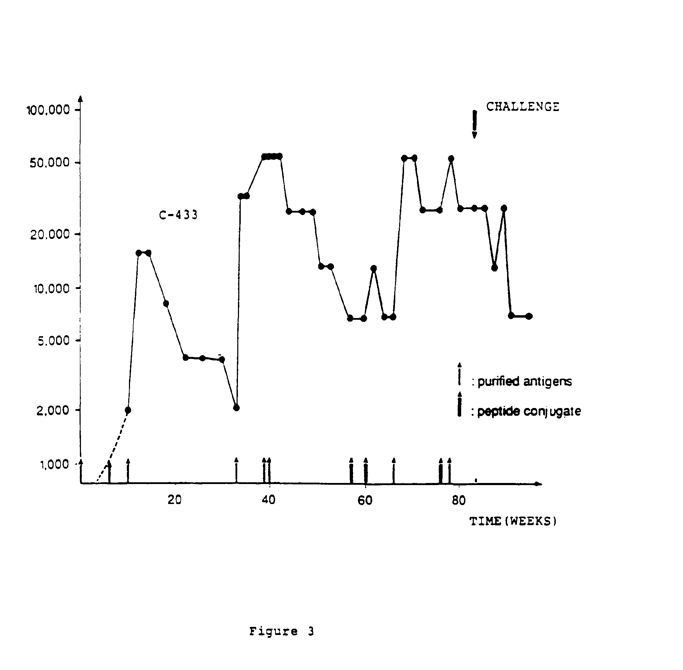

Injections of the peptide-KLH conjugate did not result in any increase in antibody levels as measured by ELISA (Fig. 3), but did result in a marked increase in neutralizing antibodies, as can be seen in Fig. 2 and in Table 3. The neutralizing antibodies were also measured using three different methods:

**Table 3**

| Induction of neutralizing antibodies in the chimpanzee ROBERT (C-433) | | | |
|---|---|---|---|
| Date after 1 st injection (weeks) | Level of neutralizing antibodies measured by method | | |
| | A | B | C |
| 0 | 200 | 64 | 200 |
| 3 | 200 | | 200 |
| 8 | >800 | 256-512 | >1600 |
| A: 90% inhibition of syncytia in MT4 cells | | | |
| B: 90% inhibition of syncytia in CEM-SS cells | | | |
| C: 75% inhibition of immunofluorescence in H9 cells | | | |

Robert was then challenged in parallel with FUNFACE, by the intravenous inoculation of 100 TCID50 of the same stock 040 of HIV-1 virus from NCI as in the previous example. Here again, total protection against infection appears to have been obtained as judging from the absence of virus in the animal's lymphocytes and the negativity of the PCR six months after challenge and by the absence of anti-p25gag and anti-p27nef antibodies, as well as the absence of anamnestic anti-HIV response as measured by ELISA or by Western blot six months after challenge. Table 4 shows the same absence of anamnestic effect on the anti-gp160 and anti-BRU neutralization epitope.

**Table 4**

| Fate of anti-gp160 and anti-major BRU neutralization epitope antibodies after challenge injection of ROBERT | | | | | |
|---|---|---|---|---|---|
| ELISA titer on date indicated | | | | | |
| Antigen | day of challenge | +1 month | +2 months | +3 months | +4 months |
| gp160 | 545,000 | 421,000 | 200,000 | 95,000 | 32,000 |
| BRU peptide | 9,000 | 6,000 | 3,000 | 3,000 | 4,000 |

### EXAMPLE 3: Immunization of a chimpanzee with gp160env and p18gag of HIV-1 antigens; amplification with HIV-1 env peptides not coupled to a carrier molecule.

Three chimpanzees were used in this experiment: the chimpanzees JOJOTOO (499), IRA (151) and HENRY II (531).

The first, JOJOTOO, received three injections, at one month intervals, of 120-150 µg of gp160env and p18gag, purified as described above, and mixed with Syntex adjuvant. This first series of injections was followed by three boosters of the same antigen given at weeks 33, 38, and 40, and a final booster at 14 months. These injections resulted in the appearance of a high antibody level detectable by Western blot and by ELISA starting immediately after the first three injections, although the level of neutralizing antibodies was relatively low, as described below.

The second chimpanzee, IRA, was immunized with 10⁸ PFU of each of the four recombinant vaccinia virus stocks expressing, respectively, gp160env, p55gag, p27nef, and p23vif of HIV-1 BRU. These inoculations given by the intradermal route, did not lead to the appearance of any neutralizing antibody, but a barely significant level (≦1:200) of antibody was detectable by Western blot or by ELISA. Chimpanzee IRA was then rested for two years.

The fourth chimpanzee, HENRY II, was naive in regard to contact with HIV or SIV antigens before the day of the experiment.

On that day the three animals described above were injected intramuscularly with a cocktail composed of 21 synthetic peptides, corresponding to the 21 sequences of the major neutralization epitope (loop V3) of HIV-1 published in Myers et al. (1989), in the amount of 50 µg per peptide, in the presence of Syntex adjuvant. Each of the peptides had a cysteine at the N-terminal position and another at the C-terminal, and thus represented the entire V-3 loop of a given isolate (amino acids 296-331 of the BRU isolate and corresponding amino acids according to the alignment of Myers et al. (1989)). The animals were reinjected with the same mixture, respectively, 1 and 2 months after the first injection. This immunization with the mixture of peptides (1.05 mg per injection) was followed in JOJOTOO with a significant anamnestic response directed against the gp160 of the BRU isolate and against its major neutralization epitope, as measured by ELISA and by using purified gp160 BRU or BRU peptide as antigen (Tables 5 and 6).

**Table 5**

| Induction of anti-gp160 BRU antibodies in response to the injection of a cocktail of free peptides corresponding to 21 sequences of the HIV-1 neutralization epitope (ELISA titer: anti-gp160 BRU) | | | | |
|---|---|---|---|---|
| Chimpanzee | Time | | | |
| | 1st injection (time 0) | 2nd injection (1 month) | 3rd injection (2 months) | 4th injection (3 months) |
| JOJOTOO (499) | 300,000 | 450,000 | 2,500,000 | 700,000 |
| IRA (151) | Negative | ND | 13,000 | 7,000 |
| HENRY II (531) | Negative | ND | Negative | Negative |
| ND: not determined | | | | |

**Table 6**

| Induction of BRU anti-neutralization epitope antibodies in response to the injection of a cocktail containing 21 peptides (ELISA anti-BRU titer) | | | | |
|---|---|---|---|---|
| Chimpanzee | Time | | | |
| | 1st injection (time 0) | 2nd injection (1 month) | 3rd injection (2 months) | 4th injection (3 months) |
| JOJOTOO (499) | 6,000 | 10,000 | 380,000 | 200,000 |
| IRA (151) | Negative | ND | 4,000 | 2,000 |
| HENRY II (531) | Negative | ND | Negative | Negative |
| ND: not determined | | | | |

The titers obtained in IRA remained very low, and they were completely negative in HENRY II. These results clearly illustrate the priming effect on the immune response resulting from pre-immunization with gp160.

The increase in the anti-peptide and anti-gp160 titer in JOJOTOO was, however, not accompanied by a marked increase in the anti-HIV ELISA titer, as can be seen (Table 7) by using a commercial diagnostic kit (ELAVIA Diagnostics Pasteur).

**Table 7**

| Anti-HIV antibody level as measured by ELAVIA | | | |
|---|---|---|---|
| Chimpanzee | Date | | |
| | Time 0 1st injection | 2 months 2nd injection | 5 months 3rd injection |
| JOJOTOO (499) | 1,000,000 | 1,600,000 | 400,000 |
| IRA (151) | Negative | 800 | 100 |
| HENRY II (531) | Negative | 200 | Negative |

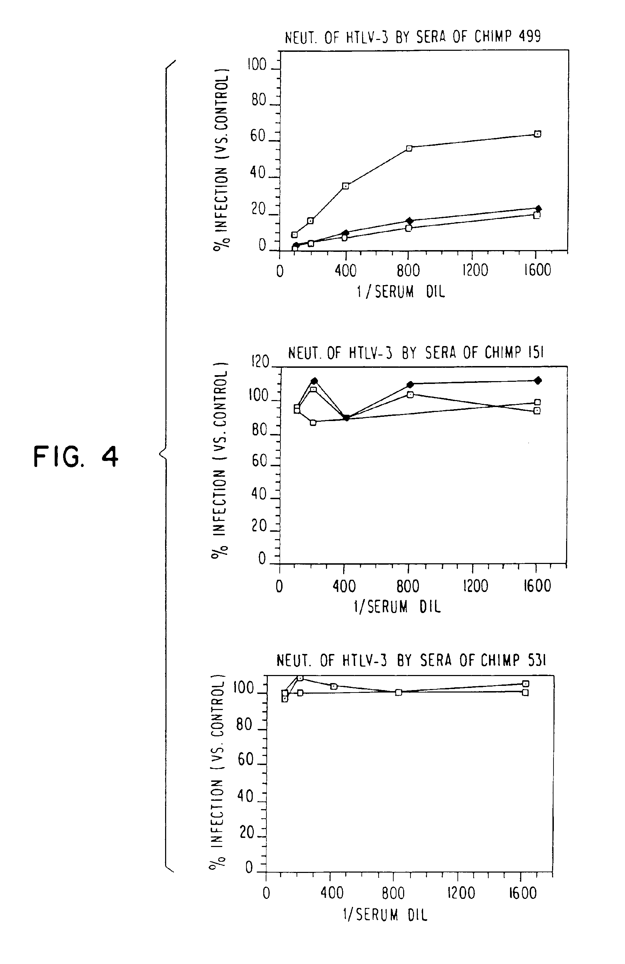

In contrast, the injections of the mixture of synthetic peptides corresponding to neutralization epitopes of the 21 isolates of HIV-1 were followed by a very clear increase in the level of antibodies neutralizing the BRU isolate, as shown in Table 8 and Figure 4. It is remarkable that this increase was seen only in JOJOTOO, but not in IRA nor in HENRY II, demonstrating the specificity of the priming effect of pre-immunization with gp160 (Figure 4).

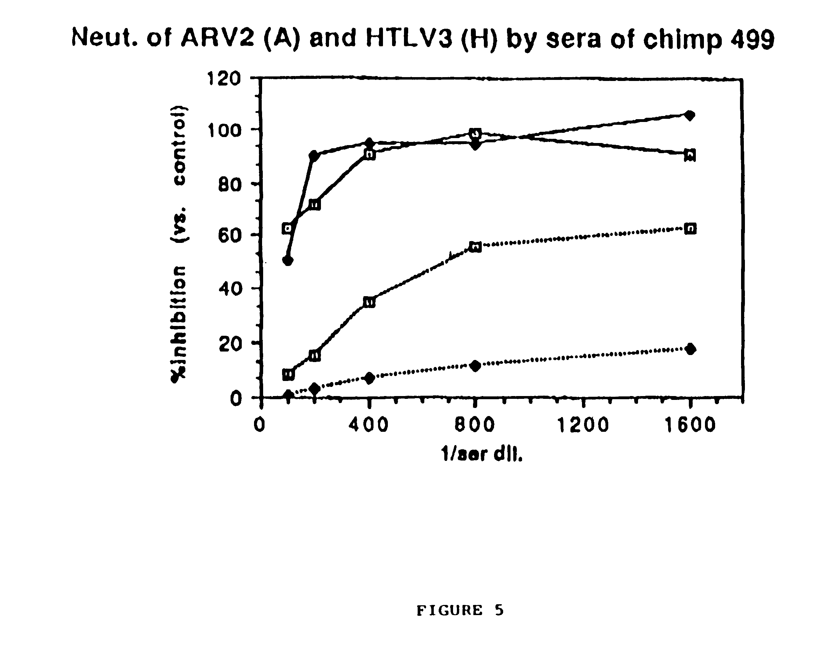

JOJOTOO's neutralizing antibody response is, moreover, specific for the BRU isolate, as can be seen in Figure 5: his serum does not neutralize the SF2 isolate (ARV-2), but only neutralizes the BRU isolate (HTLV-3=LAV1).

**Table 8**

| Level of neutralizing antibodies induced by three injections of a mixture of peptides corresponding to the 21 known sequences of the major neutralization epitope of HIV-1: 75% neutralizing titer measured on CEM-T4 cells (Method C in Table 1). | |
|---|---|
| Time | |
| 1 month before the first injection | +1 month after the third injection |
| 250 | 2,500 |

### Follow-Up Experimental Results

The most stringent test for efficacy of experimental vaccines against the human immunodeficiency virus type 1 (HIV-1) is protection of chimpanzees from infection following live virus challenge. In the study reported here, sustained high titers of neutralizing antibodies were elicited in three chimpanzees after sequential injections of different HIV-1 _{BRU} antigen preparations that included whole inactivated virus or purified recombinant proteins, followed by synthetic peptides identical to the major HIV-1 neutralizing epitope, V3. The animals were challenged intravenously with 40 chimpanzee infectious doses (equivalent to 100 50%-tissue culture infectious doses "TCID") of a stock of HIV-1 _{HTLV-IIIB·} After 6 months of follow-up, all three animals appeared uninfected by serologic and virologic criteria, including PRC analysis and failure to isolate virus from peripheral blood lymphocytes, bone marrow and lymph node tissue. Of two chimpanzees monitored for 1 year, virus was isolated initially from one animal at 32 weeks, but the second chimpanzee was virus negative by all assays through 12 months. The third animal has remained virus negative through 7 months of follow-up and also through 12 months of follow-up. These results indicate that it is possible to elicit protection against, or significantly delay infection of, HIV-1 by immunization, thus laying the foundation for development of an HIV-1 vaccine.

### Materials and Methods

Animals. Animals used in this study were adult male chimpanzees that had been used previously in hepatitis A, B and non-A and non-B experiments. The chimpanzees were maintained at LEMSIP, New York University Medical Center, in biosafety level 3 facilities. All experimental procedures were done according to institutional guidelines for containment of infectious diseases and for humane care and handling of primates (Moor-Jankowski, J. & Mahoney, C.J. (1989) J. Med. Primatol. 18, 1-26).

lmmunogens. Sucrose gradient-purified whole HIV was inactivated by incubation with 0.025% beta-propiolactone, followed by 0.025% formalin, and was shown not to contain infectious virus by failure to isolate virus from peripheral blood mononuclear cells (PBMC) of immunized chimpanzees (Girard, M., Kieny, M.P., Gluckman, J.C., Barre-Sinoussi, F., Montagnier, L. & Fultz, P. (1990) in Vaccines for Sexually Transmitted Diseases eds. Meheus, A. & Spier, R. (Butter-worth Co., Ltd., London), pp. 227-237). Recombinant gp160*env* was purified from the culture medium of BHK21 cells infected with VV-1163, a recombinant vaccinia virus expressing the gp160*env* gene modified by site-directed mutagenesis to destroy the gp120/41 cleavage site and to remove the anchor domain of gp41 (Kieny, M.P., Lathe R., Riviere, Y, Dott, K., Schmitt, D., Girard, M., Montagnier, L. & Lecocq. J.P. (1988) Prot. Engineering 2, 219-226; and Schmidt, D., Dezutter-Dambuyant, C., Hanau, D., Schmitt, D.A., Kolbe, H.V.J., Kieny, M.P., Cazenave, J.P. & Thivolet, J. (1989) Comptes Rendus Acad. Sci. Paris, 308(III), 269-275). Where indicated, the antigen was mixed with recombinant p18*gag*, *p27nef* and p23*vif* antigens that were purified from E. coli pTG2153, pTG1166 and pTG1149, respectively, as described (Guy, B., Riviere, Y, Dott, K. Regnault, A. & Kieny, M.P. (1990) Virology 176, 413-425; and Kolbe, H.V, Jaeger, F., Lepage, P., Roitsch, C., Lacaud, G., Kieny, M.P., Sabatie, J., Brown, S.W. & Lecocq, J.P. (1989) J. Chromatography 476, 99-112). Before each immunization, inactivated whole HIV (250 µg viral protein) or the purified recombinant proteins (125-150 µg each per dose) were mixed with the adjuvant SAF-1 (Allison, A.C. & Byars, N.E. (1986) J. Immunol. Methods 95, 157-168), and 2 ml of the mixtures were injected intramuscularly (IM).

An aliquot (19.8 mg) of a 25-amino acid peptide, with the sequence Y-NTRKSIRIQRGPGRAFVTIGKIGN (Putney, S.D., Matthews, T.J., Robey, W.G., Lynn, D.L., Robert-Guroff, M., Mueller, W.T., Langlois, A.L., Ghrayeb, J., Petteway, S.R., Weinhold, K.J., Fischinger, P.J., Wong-Staal, F., Gallo, R.C. & Bolognesi, D.P. (1986) Science 234, 1392-1395; Rusche, J.R., Kavaherian, K., McDanal, C., Petro, J., Lynn, D.L., Grimaila, R., Langlois, A., Gallo, R.C., Arthur, L.O., Fischinger, P.J., Bolognesi, D.P., Putney, S.D. & Matthews, T.J. (1988) Proc. Natl. Acad. Sci. U.S.A. 85, 3198-3202; and LaRosa, G.J., Davide, J.P., Weinhold, K., Waterbury, J.A., Profy, A.T., Lewis, J.A., Langlois, A.J., A.J., Dressman, G.R. Boswell, R.N., Shadduck, P., Holley, L.H., Karplus, M., Bolognesi, D.P., Matthews, T.J. Emini, E.A. & Putney, S.D. (1990) Science 249 932-935) was treated first with citraconic acid and then was coupled to 19.3 mg keyhole limpet hemocyanin (KLH) by N-terminal tyrosyl linkage using bis-diazobenzidine (pH 9.0). After the block on amino groups was removed, the peptide-KLH conjugate was dialyzed for 24 hours against PBS to remove excess free peptide. After formulation with SAF-1, immunizations with the V3 peptide-KLH conjugate (300 µg peptide per dose) were done by the IM route.

Challenge Virus. The challenge inoculum was from a stock of HIV-1 strain HTLV-IIIB (obtained from L. Arthur), which had been titrated in chimpanzees and used in other HIV vaccine challenge studies (Arthur, L.O., Bess, J.W., Waters, D.J., Pyle, S.W., Kelliher, J.C., Nara, P.L., Krohn, K.,Robey, W.G., Langlois, A.J., Gallo, R.C. & Fischinger, P.J. (1989) J. Virol. 63,5046-5053; and Berman, P.W., Gregory, T.J., Riddle, L., Nakamura, G.R., Champe, M..A., Porter, J.P., Wurm, F.M., Hershberg, R.D., Cobb, E.K. & Eichberg, J. W. (1990) Nature (London) 345, 622-625). The infectivity titer of this HIV-1 stock is considered to be 10⁴ TCID₅₀ per ml and 4 x 10³ infectious units per ml for chimpanzees. The chimpanzees were challenged IV with 1 ml of a 1:100 dilution. Aliquots of these same 1:100 dilutions were titrated in quadruplicate by twofold serial dilution and infection of 1 x 10⁵ H9 cells in 96-well microtiter plates. After incubation for 6 days, infection was scored by immunofluorescence assay. By this method, the challenge inoculum had a titer of greater than 64 immunofluorescent focus-forming units (end-point not reached) for the first aliquot and 170 for the second.

Neutralization Assay. Neutralization activity in serum samples from immunized chimpanzees was determined by inhibition of syncytia formation in CEM-SS cells, as described (Nara, P.L., Hatch, W.C., Dunlop, N.M., Robey, W.G., Arthur, L.O., Gonda, M.A. & Fischinger, P.J. (1987) AIDS Res. Human Retroviruses 3, 283-302), or inhibition of immunofluorescent foci in H9 cells.

Virus Isolation. PBMC or bone marrow cells (obtained as aspirates) from immunized and challenged chimpanzees were cultured with normal human PBMC, as described (Fultz, P.N., McClure, H.M., Swenson, R.B., McGrath, C.R., Brodie, A., Getchell, J.P., Jensen, F.C., Anderson, D.C., Broderson, J.R. & Francis, D.P. (1986) J. Virol., 58, 116-124). In some experiments, CD⁺4-enriched lymphocytes were obtained from chimpanzee PBMC by separation with magnetic beads to which were attached monoclonal antibodies specific for the CD8 cell-surface antigen (Dynabeads, Robbins Scientific). The CD⁺4-enriched cells were stimulated 2 days with concanavalin A (10 µg/ml) before being cultured alone or cocultured with phytohemagglutinin (PHA)-stimulated normal human PBMC in RPMI-1640 medium with 10% fetal bovine serum, glutamine, gentomicin and recombinant interleukin-2 (8 units/ml; Boehringer Mannheim). Lymph node tissue obtained by biopsy was minced with scissors and cultured with human PBMC. All cultures were maintained and monitored for reverse transcriptase activity for 6 weeks before being discarded.

Polymerase Chain Reaction (PCR). Both single- and double-round (nested) PCR were performed periodically with PBMC or lymph node cells from challenged chimpanzees. Single-round PCR was as described (Laure, F., Rouzioux, C., Veber, F., Jacomet, C., Courgnaud, V., Blanche, S., Burgard, M., Griscelli, C. & Brechot, C. (1988) Lancet 2, 538-541). Briefly, 2 µg DNA were used with 2 units *Taq*-1 DNA polymerase for 40 cycles at 94°C, 55°C, and 72°C (1 min each). Two primer pairs were used: one corresponded to nucleotides 2393-2417 and 2675-2700, encoded by the *pol* gene, and the other corresponded to nucleotides 5367-5385 and 5694-5711, encoded by the *tat* gene. To show specificity of the PCR, amplified DNA fragments were hybridized with [³²P]-labeled internal *pol* and *tat* gene probes. The positive control consisted of DNA from the 8E5 cell line persistently infected with LAV-1. For nested PCR, the primers for the first round of PCR, performed as described (Mullis, K.B. & Faloona, F.R. (1987) Methods Enzymol. 155, 335-350) were: 5'-GCTTCTAGATAATACAGTAGCAACCCTCTATTG-3', corresponding to a 3-base clamp sequence, an *Xbal* restriction site and nucleotides 1025-1048 of the HXB2 genome, and: 5'-GTCGGCCTTAAAGGCCCTGGGGCTTGTTCCATCTATC-3', corresponding to a 3-base clamp sequence, a *Notl* restriction site and nucleotides 5573-5553 of the HXB2 genome. From the first round, 2.5 µl of the product was reamplified with primers SK145 and SK150 (Kwok, S. & Kellogg, D.E. (1990) in PCR Protocols: A Guide to Methods and Applications: eds. Innis, M.A., Gelfand, D.H., Sninsky, J.J. & White T.J. (Academic Press, Inc., San Diego, CA) pp. 337-347), over a region from nucleotides 1366 to 1507 on the HXB2 genome.

### Immunization Regimens (Table 9)

**TABLE 9**

| Immunization regimens of chimpanzees with various HIV-1 antigens | | | | | | | |
|---|---|---|---|---|---|---|---|
| Animal | Recombinant VV-1139 | Inactivated HIV | Recombinant antigens | | | | V3 peptide |
| | | | gp160 | gag | nef | vif | |
| C-433 | + | + | + | + | + | + | + |
| C-339 | - | + | + | - | - | - | + |
| C-499 | - | - | + | + | - | - | + |

For C-433 and C-339, times of immunizations and virus challenge were calculated from the time that C-433 received its first immunization with VV-1139, which is considered week 0. Chimpanzee C-433 was first immunized with a recombinant vaccinia virus, VV-1139, that expresses a non-cleavable version of the HIV.1_{BRU} gp160*env* antigen (Kieny, M.P., Lathe R., Riviere, Y, Dott, K., Schmitt, D., Girard, M., Montagnier, L. & Lecocq. J.P. (1988) Prot. Engineering 2, 219-226). VV-1139 was administered on weeks 0, 8 and 21 by scarification on the upper back with a two-pronged needle (2 x 10⁸ PFU per inoculum). At week 27, PBMC from C-433 were stimulated with PHA, cultured in medium containing IL-2 and then infected with VV-1139 at a multiplicity of infection of 7. Following culture for an additional 16 hours, the PBMC were fixed with 0.8% paraformaldehyde and reinjected into C-433 by the IV route (Zagury, D., Bernard, J., Cheynier, R., Desportes, I., Leonard, R., Fouchard, M., Reveil, B., Ittele, F.D., Lurhama, Z., Mbayo, K., Wane, J., Salaun, J.J., Goussard, B., Dechazal, L., Burny, A., Nara, P. & Gallo, R.C. (1988) Nature (London) 322, 728-731). At weeks 48, 54, 58, 81, 86, 88, 114 and 124, C-433 was inoculated IM with mixtures of purified gp160*env*, p18*gag*, *p27nef* and p23vif (125-250 µg each per dose) formulated with SAF-1.

Chimpanzee C-339 was first immunized on week 33 by IM injection of inactivated HIV (125 µg viral protein) mixed with SAF-1 (1 mg threonyl muramyl dipeptide), followed by booster inoculations on weeks 37, 41, 62 and 124. C-339 was then inoculated with purified gp160*env* only (125 µg per dose) on weeks 66, 74, 81, 85 and 87. The V3 peptide (300 µg peptide per dose) was administered IM on weeks 105, 108 and 126.

C-339 and C-433 were challenged on week 131 with 100 TCID₅₀ of HIV-1_{HTLV IIIB}. C-449 was inoculated IM with a mixture of gp160*env*, p18*gag* and SAF-1 on weeks 0, 6, 10, 33, 38, 66 and 76. (Note: week 0 for C-499 corresponds to week 48 for C-433 and C-339.) A mixture of 21 free V3 peptides (100 µg each per dose) was administered IM with SAF-1 on weeks 79, 83, 87 and 102. C-499 and C-087, a naive control, were challenged on week 106 and 100 TCID₅₀ of HIV_{HTLV-IIIB}.

### Results

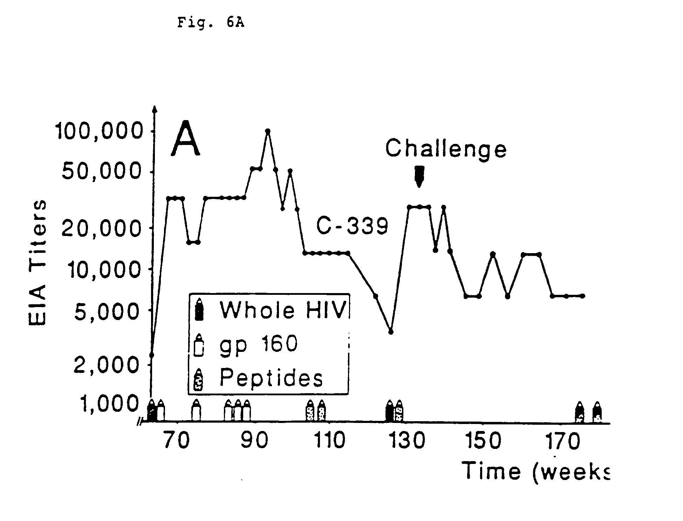

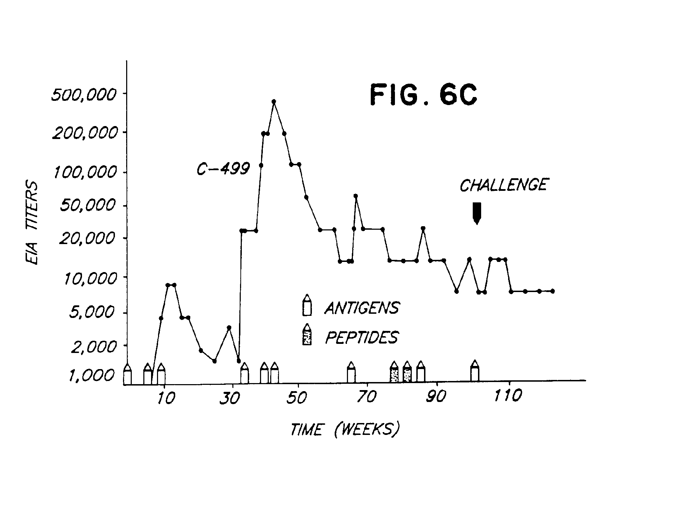

Immunization of chimpanzee C-339 with formalin- and beta-propiolactone-inactivated whole HIV mixed with the adjuvant SAF-1 resulted in high titers of antibodies to *gag-* and *env*-encoded proteins, as measured by enzyme immunoassay (EIA), a low neutralizing antibody response, and no detectable cell-mediated immune response. In an effort to enhance immune responses, C-339 was immunized with purified recombinant gp160*env*. Following one intradermal inoculation of gp160*env* with BCG in multiple sites on the chest, C-339 was given four successive IM injections of the same antigen formulated with SAF-1. Total EIA antibody and neutralizing antibody titers were determined periodically; however, during the course of immunization, both remained unchanged and decreased rapidly after the injections were discontinued (Figure 6A).

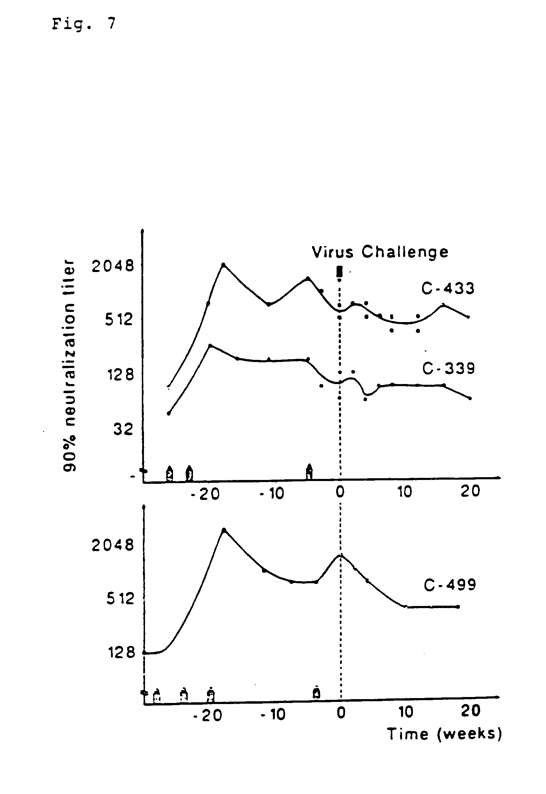

In HIV-infected persons, the majority of HIV-neutralizing antibodies are directed against the third hypervariable region of the external envelope glycoprotein, termed the V3 loop (Putney, S.D., Matthews, T.J., Robey, W.G., Lynn, D.L., Robert-Guroff, M., Mueller, W.T., Langlois, A.L., Ghrayeb, J., Petteway, S.R., Weinhold, K.J., Fischinger, P.J., Wong-Staal, F., Gallo, R.C. & Bolognesi, D.P. (1986) Science 234, 1392-1395; Rusche, J.R., Kavaherian, K., McDanal, C., Petro, J., Lynn, D.L., Grimaila, R., Langlois, A., Gallo, R.C., Arthur, L.O., Fischinger, P.J., Bolognesi, D.P., Putney, S.D. & Matthews, T.J. (1988) Proc. Natl. Acad Sci. U.S.A. 85, 3198-3202; and LaRosa, G.J., Davide, J.P, Weinhold, K., Waterbury, J.A., Profy, A.T., Lewis, J.A., Langlois, A.J., A.J., Dressman, G.R. Boswell, R.N., Shadduck, P., Holley, L.H., Karplus, M., Bolognesi, D.P, Matthews, T.J. Emini, E.A. & Putney, S.D. (1990) Science 249 932-935). Antibodies to epitopes within the loop abrogate virus infectivity, probably by preventing fusion of the viral envelope to the target cell membrane. Neutralizing antibodies to V3 epitopes can, in fact, be added as long as 40 to 60 minutes after virus binds to the cell and still prevent infection (Nara, P.L., (1989) in Vaccines 89, eds. Lerner, R.A., Ginsberg, H., Chanock, R.M. & Brown, F. (Cold Spring Harbor Laboratory, Cold Spring Harbor, NY) pp. 137-144). Therefore, to determine whether immunization with the V3 loop would boost neutralizing antibody titers, C-339 was injected with an oligopeptide of 25 amino acids, having the V3 sequence of HIV-1_{BRU(IIIB)}, cross-linked to KLH and formulated with SAF-1. No change in EIA titer was observed (Figure 6A), but a significant increase in neutralizing antibody titers, which were sustained for several months, was obtained following the second immunization at week 108 (Figure 7A).

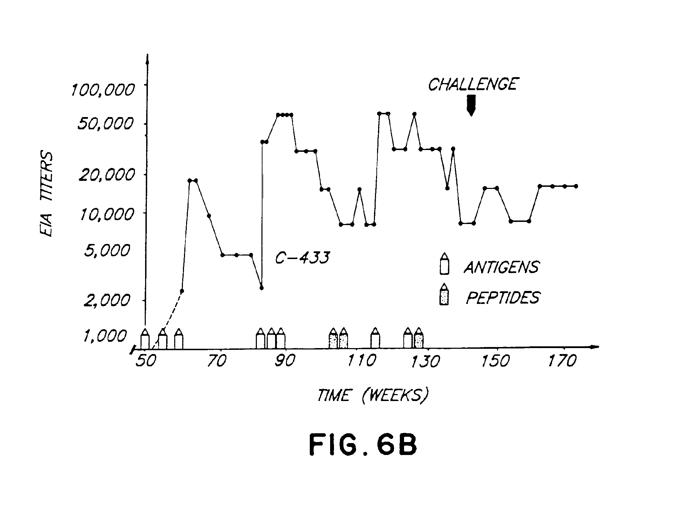

Another chimpanzee, C-433, that had been primed by vaccination with VV-1139 (Kieny, M.P., Lathe R., Riviere, Y, Dott, K., Schmitt, D., Girard, M., Montagnier, L. & Lecocq. J.P. (1988) Prot. Engineering 2, 219-226), was immunized repeatedly with 125-250 µg each of recombinant soluble gp160*env*, p18*gag*, p27*nef* and *p23vif* (Table 1). The anti-HIV antibody response induced by this regimen was clearly transient, with titers rising sharply after each booster injection and then decreasing rapidly (Figure 6B). The neutralizing antibody and EIA titers of C-433 fluctuated in parallel. Finally, C-433 was injected with the same V3 peptide-KLH conjugate as C-339, according to the same immunization protocol. Neutralizing antibody titers increased significantly after the second injection of the V3-peptide conjugate and remained high thereafter (Figure 7A); a third immunization 4 months later (week 126) elicited no change in titers.

At the time C-433 first received the purified recombinant proteins (48 weeks), a third chimpanzee, C-499, received an IM injection of purified gp160*env* and p18*gag* formulated with SAF-1. C-499 received six booster innoculations of the same antigens, followed by a series of four injections of a mixture of 21 free (unconjugated) V3 peptides (Myers, G. (1990) in Human Retroviruses and AIDS, eds. Myers, G., Josephs, S.F., Wong-Staal, F., Rabson, A.B., Smith, T.F. & Berzofsky, J.A. (Los Alamos National Laboratory, Los Alamos, NM) in SAF-1. As with C-339 and C-433, C-499's EIA titers declined rapidly after immunization with the purified HIV antigens, and there was no detectable effect of the V3 peptides on EIA titer. There was, however, a significant increase in neutralizing antibody titers (to > 2000) following the V3 peptide inoculations (Figure 7B).

**Challenge *with* Infectious HIV.** Because sustained neutralizing antibody titers were achieved, chimpanzees C-433, C-339 and C-499 were challenged by IV inoculation of 100 TCID₅₀ (40 chimpanzee infectious doses) of HIV-1. At the time of challenge, 50% neutralization titers by an immunofluorescence inhibition assay were 1:2000, 1:280-350 and 1:2000, and 90% neutralization titers by a syncytia-inhibition assay (Nara, PL., Hatch, W.C., Dunlop, N.M., Robey, W.G., Arthur, L.O., Gonda, M.A. & Fischinger, P.J. (1987) AIDS Res. Human Retroviruses 3, 283-302) were 1:512-1024, 1:128 and 1:1024 for chimpanzees C-433, C-399 and C-499, respectively. Because immunization of C-499 was initiated at a different time from the other two animals, challenge of C-499 occurred 6 months after that of C-399 and C-433, but was done at the same time as that of a naive control animal, C-087. Virus was isolated from C-087's PBMC at 2 weeks post-inoculation (PI) as well as at all subsequent times, showing that a 1:100 dilution of the HIV-1 stock readily infected chimpanzees under our conditions.

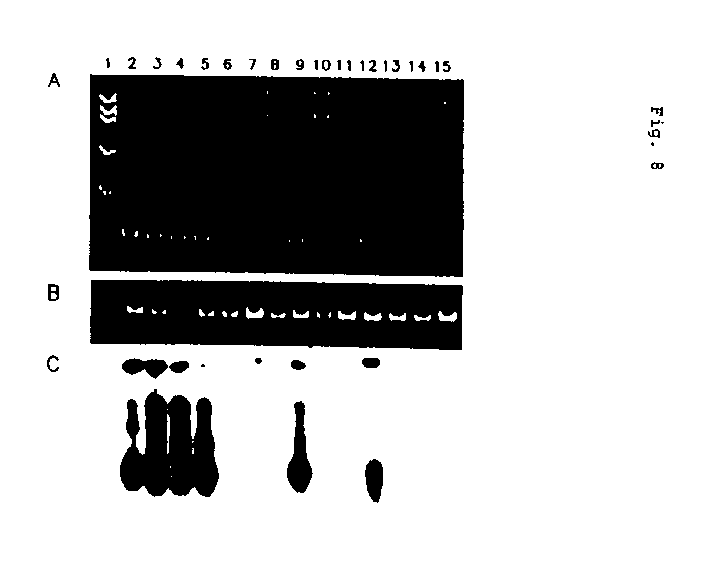

Attempts to **Isolate HIV** from **Immunized and Challenged** Chimpanzees. At various times after challenge with HIV-1, three methods were used to assess the infection status of the immunized animals. First, attempts to detect HIV sequences in lymphoid cells by PCR were made periodically (Laure, F., Rouzioux, C., Veber, F., Jacomet, C., Courgnaud, V., Blanche, S., Burgard, M., Griscelli, C. & Brechot, C. (1988) Lancet 2, 538-541; Mullis, K.B. & Faloona, F.A. (1987) Methods Enzymol. 155, 335-350; and Kwok, S. & Kellogg, D.E. (1990) in PCR Protocols: A Guide to Methods and Applications: eds. Innis, M.A., Gelfand, D.H., Sninsky, J.J. & White T.J. (Academic Press, Inc., San Diego, CA) pp. 337-347). DNA samples obtained from PBMC of the three chimpanzees at 3 weeks and 3 and 6 months after challenge were tested. Bands with the expected electrophoretic mobility were detected in DNA from a control HIV-infected chimpanzee, but not in PBMC from the vaccinated and challenged animals or from a control naive animal (data not shown). At 6 months after challenge, nested sets of primers were used to perform PCR analysis on DNA from both PBMC and lymph node tissue of the challenged and control chimpanzees (Mullis, K.B. & Faloona, F.A. (1987) Methods Enzymol. 155, 335-350). This technique is more sensitive than standard PCR, and in these experiments (repeated at least seven times on all samples), approximately one molecule of viral DNA was found to produce a strong signal when present in 1.5 x 10⁵ cell-equivalents of DNA. All PBMC and lymph node samples were consistently negative except those from a previously infected chimpanzee, which were always positive (Figure 8). Thus, at 6 months after challenge, viral DNA was not present in PBMC and lymph node tissues at a frequency greater than one copy per 10⁶ cells.

Second, at weeks 2, 4, 6 and 8, and at monthly intervals thereafter, attempts were made to isolate virus from PBMC by cocultivation of the chimpanzees' PBMC with lymphocytes obtained from normal humans (Fultz, P.N., McClure, H.M., Swenson, R.B., McGrath, C.R., Brodie, A., Getchell, J.P., Jensen, F.C., Anderson, D.C., Broderson, J.R. & Francis, D.P. (1986) J. Virol., 58, 116-124). Because CD8⁺ cells have been shown to suppress virus replication not only in HIV-infected humans (Walker, C.M., Moody, D.J., Stites, D.P. & Levy, J.A. (1986) Science 234, 1563-1566; and Tsubota, H., Lord, C.I., Watkins, D.I., Morimoto, C. & Letvin, N.L. (1989) J. Exp. Med. 169, 1421-1434) and chimpanzees (PN.F., unpublished data), but also in SIV-infected macaques (Tsubota, H., Lord, C.I., Watkins, D.I., Morimoto, C. & Letvin, N.L. (1989) J. Exp. Med. 169, 1421-1434), in some experiments chimpanzee PBMC were depleted of CD8⁺ lymphocytes before cultures were established. In contrast to virus recovery from the control animal, C-087, virus was not recovered from either total PBMC or CD4⁺-enriched cells from C-339, C-433, or C-499 at any time during the first 6 months of follow-up. At 6 months PI, inguinal lymph node biopsies were performed on all animals as well as on uninfected and HIV-infected control chimpanzees. Upon cocultivation with normal human PBMC, virus was recovered from the lymph node of the infected control, but not from those of the immunized and challenged chimpanzees (data not shown). Despite the fact that all attempts to detect virus during the first 6 months after challenge had failed, virus was isolated from C-433 by cocultivation of PBMC obtained at 32 weeks and thereafter and of bone marrow obtained 37 weeks after challenge.

Lastly, the challenged animals were monitored for possible seroconversion to HIV antigens that were not included in their immunization regimens. Immunoblot analysis (Diagnostic Pasteur) showed that C-433 and C-499, which had been immunized with, among other antigens, p18*gag* but not p25*gag*, did not seroconvert to p25 during 7 months follow-up; however, at 32 weeks (7½ months) PI, a faint p25 band was observed on immunoblots for C-433, which increased in intensity with succeeding serum samples (Figure 9). For C-339, which had been immunized with whole inactivated HIV, there were no detectable increases in EIA antibody titers or in apparent levels of antibodies to any HIV-specific proteins (Figure 9). Also, using purified antigens in immunoblot assays, no antibodies to the *vif* or *nef* proteins were detected in serum from C-339 during 12 months follow-up.

The results presented here, as well as those reported by Berman and colleagues (Berman, P.W., Gregory, T.J., Riddle, L., Nakamura, G.R., Champe, M..A., Porter, J.P., Wurm, F.M., Hershberg, R.D., Cobb, E.K. & Eichberg, J. W. (1990) Nature (London) 345, 622-625), clearly show that it is possible to elicit a protective immune response in chimpanzees with various HIV-1 antigens. It has been shown that C-499 was protected against establishment of HIV infection, at least through 7 months follow-up, that C-339 was protected for 1 year, and that C-433 was protected partially, as evidenced by the 7-month delay in appearance of virus. It is possible, however, that C-433 also might have been fully protected if the challenge dose had been the same as that used by others (Berman, P.W., Gregory, T.J., Riddle, L., Nakamura, G.R., Champe, M..A., Porter, J.P., Wurm, F.M., Hershberg, R.D., Cobb, E.K. & Eichberg, J. W. (1990) Nature (London) 345, 622-625), which was fourfold lower than the dose used herein. Protection was demonstrated by: (1) failure to recover virus from PBMC during monthly attempts and from lymph node tissue at 6 months PI: (ii) negative hybridization signals in PCR analysis of DNA from PBMC at various intervals and from lymph nodes at 6 months PI, and (iii) the absence of antibody responses that normally follow a primary HIV infection or that are characteristic of anamnestic responses in previously vaccinated and challenged animals (Berman, P.W., Groopman, J.E., Gregory, T., Clapham, P.R., Weiss, R.A., Ferriani, R. Riddle, L., Shimasaki, C., Lucas, C., Lasky, L.A. & Eichberg, J.W. (1988) Proc. Natl. Acad. Sci. U.S.A. 85 5200-5204; Arthur, L.O., Bess, J.W., Waters, D.J., Pyle, S.W., Kelliher, J.C., Nara, PL., Krohn, K.,Robey, W.G., Langlois, A.J., Gallo, R.C. & Fischinger, P.J. (1989) J. Virol. 63,5046-5053; Girard, M., Kieny, M.P., Gluckman, J.C., Barre-Sinoussi, F., Montagnier, L. & Fultz, P. (1990) in Vaccines for Sexually Transmitted Diseases eds. Meheus, A. & Spier, R. (Butterworth Co., Ltd., London), pp. 227-237).

That C-433 appeared to be protected for 7 months, but actually was infected from time of challenge, despite repeatedly negative results for virus isolation and detection by PCR, is worrisome and underscores the fact that HIV can be sequestered such that it defies detection by both virologic and serologic criteria. A similar occurrence was reported (Desrosiers, R.C., Wyand, M.S., Kodama, T., Ringler, D.J., Arthur, L.O., Sehgal, P.K., Letvin, N.L., King, N.W. & Daniel, M.D. (1989) Proc. Natl. Acad Sci. U.S.A. 86 86, 6353-6357) for a macaque immunized with inactivated whole virus and then challenged with infectious SIV In that study, virus was not recovered initially until 32 weeks and an anamnestic response was not observed until 39 weeks after challenge. The observation in natural HIV infections that persons remained seronegative by conventional tests for extended times, but HIV was detected by PCR or virus isolation (Ranki, A., Valle, S.L., Krohn, M., Antonen, J., Allain, J.P., Leuther, M., Franchini, G. & Krohn, K. (1987) Lancet 2, 589-593; and Jehuda-Cohen, T., Slade, B.A., Powell, J.D., Villinger, F., De, B., Folks, TM., McClure, H.M., Sell, K.W. &Ahmed-Ansari, A. (1990) Proc. Natl. Acad. Sci. U.S.A. 87, 3972-3976), suggests that high-risk individuals, such as sexual partners of HIV-infected persons, possibly could be infected despite negative serologic, virologic or PCR analyses.

In view of the complex regimen of immunization undergone by the three chimpanzees, it is difficult to determine which of the many antigens and/or antigen formulations were instrumental in eliciting partial protection. C-339 was immunized successively with inactivated HIV, purified gp160, and the V3 peptide-KLH conjugate. C-433 was immunized first with a vaccinia virus-gp160*env* recombinant, then with a mixture of purified *env,* p18*gag*, *nef* and *vif* antigens, and finally with the V3 peptide-KLH conjugate. The simplest immunization regimen was that of C-499; it consisted of purified gp160*env* and p18*gag* followed by unconjugated V3 peptides. The antigens that were common to the three animals were gp160*env*, p18*gag* and the V3 peptide, but their relative importance remains to be determined. Adequate protection might require multiple antigenic determinants found on more than one viral protein, and/or multiple presentations of the same antigenic determinant.

It is of interest that previously tested prototype vaccines (Berman, P.W., Groopman, J.E., Gregory, T, Clapham, P.R., Weiss, R.A., Ferriani, R. Riddle, L., Shimasaki, C., Lucas, C., Lasky, L.A. & Eichberg, J.W. (1988) Proc. NaCl. Acad. Sci. U.S.A. 85 5200-5204; Arthur, L.O., Bess, J.W., Waters, D.J., Pyle, S.W., Kelliher, J.C., Nara, P.L., Krohn, K.,Robey, W.G., Langlois, A.J., Gallo, R.C. & Fischinger, P.J. (1989) J. Virol. 63,5046-5053; Girard, M., Kieny, M.P., Gluckman, J.C., Barre-Sinoussi, F., Montagnier, L. & Fultz, P. (1990) in Vaccines for Sexually Transmitted Diseases eds. Meheus, A. & Spier, R. (Butterworth Co., Ltd., London), pp. 227-237; and Hu, S.L. Fultz, P.N., McClure, H.M., Eichberg, J.W., Thomas, E.K., Zarling, J., Singhal, M.C., Kosowski, S.G., Swenson, R.B., Anderson, D., C. & Todaro, G. (1987) Nature (London) 328, 721-723) that did not elicit significant titers of neutralizing antibodies in chimpanzees were not effective in preventing experimental infection of the animals. The observation that sustained neutralizing antibody titers were reached in C-339 and C-433 after two injections of the V3 peptide-KLH conjugate and in C-499 after three injections of V3 peptides (Figure 7), suggests that V3 night be seen differently by the chimpanzee immune system when presented as a peptide than when presented as part of the gp160/120*env* molecule. We have found by immunoaffinity chromatography that virtually all HIV-neutralizing activity in the serum of the protected chimpanzees could be adsorbed by the V3 peptide (unpublished data of A.P.). The booster inoculations of the V3 peptide(s) might explain why immunization with gp160 resulted in protection of chimpanzees in the subject experiments, but not in those reported by Berman et al. (Berman, P.W., Gregory, T.J., Riddle, L., Nakamura, G.R., Champe, M..A., Porter, J.P., Wurm, F.M., Hershberg, R.D., Cobb, E.K. & Eichberg, J. W. (1990) Nature (London) 345, 622-625). In this latter study, two chimpanzees were protected after immunization with gp120, and these animals had three-to four-fold higher titers to the principal neutralizing determinant (PND) found in the V3 loop than the two animals not protected from infection.

The question of whether the protection observed in the present experiment wan due solely to neutralizing antibodies or whether other immune mechanisms were involved remains unanswered. At time of challenge, antibody-dependent cellular cytotoxic activity was detected in the serum of C-339, but not in that of the other two chimpanzees. HIV-specific proliferative responses to the soluble proteins p18*gag*, gp160*env*, and p27nef (Bahraoui, E., Yagello, M., Billaud, J.N., Sabatier, J.M., Guy, B., Muchmore, E., Girard, M. & Gluckman, J.C. (1990) AIDS Res. Human Retroviruses 6, 1087-1088; and Van Eendenburg, J.P., Yagello, M., Girard, M., Kieny, M.P., Lecocq, J.P., Muchmore, E., Fultz, P.N., Riviere, Y, Montagnier, L. & Gluckman, J.C. (1989) AIDS Res. Human Retroviruses 5, 41-50) were detected in PBMC from C-433 both before and after virus challenge, but not in PBMC from C-339. Interestingly, after immunization with the V3-KLH conjugate, C-433 displayed a sustained, strong T-helper cell reactivity to the V3 peptide, while C-339 had only a weak response. The responses of C-449 are currently under study. Repeated attempts to detect cytotoxic T lymphocytes (CTL) in PBMC of the vaccinated chimpanzees before, on the day of, and after challenge have failed. It appears, therefore, that the observed protection did not correlate with the T-helper cell or CTL activity.

The results presented here indicate that HIV vaccines can induce protection against virus infection. The high neutralizing antibody response induced by the V3 peptide was type specific; serum from the vaccinated animals at time of challenge neutralized the more diverse HIV-1 isolates RF and MN only marginally (unpublished data). Therefore, it will be necessary to design a vaccine that will induce high titers of neutralizing antibodies to the many HIV variants, but the recent identification (LaRosa, G.J., Davide, J.P., Weinhold, K., Waterbury, J.A., Profy, A.T., Lewis, J.A., Langlois, A.J., A.J., Dressman, G.R. Boswell, R.N., Shadduck, P., Holley, L.H., Karplus, M., Bolognesi, D.P., Matthews, T.J. Emini, E.A. & Putney, S.D. (1990) Science 249 932-935) of PND sequences with which a majority of sera from HIV-infected persons react may make this less formidable than previously thought. The apparent success in protecting two chimpanzees and suppression of virus for an extended period in a third animal justify further efforts to develop an HIV vaccine, with the expectation that it will provide long-lasting protective immunity in humans.

Further studies were conducted to ascertain the validity of the dual immunization procedure (priming with gp160 followed by boosting with synthetic peptides with the sequence of the V3 loop of gp120); to compare 3 adjuvants : Al(OH)₃, the Syntex adjuvant, SAF-1, and incomplete Freund adjuvant (IFR); and to test an accelerated schedule of immunization: gp160 at 0 and 1 month, the V3 peptide at 3 and 4 months, and both gp160 and V3 as a last boost at 6 months.

The experiment was carried out in Rhesus macaques (4 animals per lot) using 100 µg of gp160 BRU for priming and a mixture of 200 µg each of V3-BRU (gp120 amino acid residues 302-335) and V3-MN (same residues) for boosting. The animals were bled at monthly intervals and anti-V3 and anti-gp antibody (Ab) titers were determined by ELISA. Neutralizing Ab titers were determined by the inhibition of immunofluorescent foci formation assay

Anti-gp160 Ab were measured by ELISA using plaques coated with purified gp160 BRU. A fast anti-gp160 Ab response was observed in the 3 groups of animals (Fig. 10), but the response to the antigen in the groups with IFA and SAF-1 was from 5 to 10 fold higher than that in the group with alum. Injection of V3 peptides had no effect on anti-gp160 titers. Titers were boosted several fold upon recall injection of gp160 at 6 months, but again, the group with alum had a 2-8 fold lower response than the other 2.

Anti-V3 Ab were measured by ELISA using plaques coated with the BRU peptide. The response to V3 was clearly biphasic in all groups, with a strong booster effect seen upon injection of the V3 peptide at 3 months (Fig. 11). Thus, anti-V3 titers increased 10 fold between months 3 and 4 and then plateaued, confirming the remarkable booster effect of a V3 peptide injection in gp160-primed animals. This was observed irrespective of the adjuvant used in the experiment.

The initial response to V3, measured at month 3, was, however, 5-6 fold higher in the SAF-1 and IFA groups than in the group with alum. The final anti-V3 titers were altogether about 10 fold higher in the former 2 groups than in the latter. A two-step immunization schedule can be defined as follows:
priming: gp160 at 0 and 1 month
boosting: V3 peptides at 3 months
second boosting: gp160 + V3 peptides at 6 months.

The second boost can be placed at a later time, such as 12 months, to increase further the anamnestic response.

All pre-immune sera were negative for neutralizing Ab. Titers of neutralizing Ab measured at one month after the second boost (month 7) were the following:

| Monkeys | Adjuvant | | |
|---|---|---|---|
| | Al(OH)₃ | SAF-1 | IFA |
| 1 | 60 | 140 | > 450 |
| 2 | neg | 135 | 340 |
| 3 | 123 | > 450 | 292 |
| 4 | neg | > 450 | 440 |

Here again, there was a definite advantage in using SAF-1 or incomplete Freund adjuvant over using alum, although the relative difference in titers was somewhat less pronounced between the various groups.

In conclusion, a fast 2-step anti-HIV immunization schedule for primates is able to induce high anti-V3, high anti-gp160, and high neutralizing Ab responses. This schedule includes:

gp        gp                V3      V3          gp + V3
0         1                 3       4           6

An alternative to that schedule could be:

gp          gp          V3          gp + V3
0           1           3           6

There is an advantage in using the Syntex adjuvant SAF-1 or incomplete Freund adjuvant rather than Alum [Al(OH)₃], as final Ab titers are from 5 to 15 fold higher with the former 2 adjuvants us compared to the latter.

### REFERENCES

1. Koff, W.C. & Fauci, A.S. (1989) AIDS 3(S1), S125-S129.
2. Ada, G.L. (1989) Nature (London) 339, 331-332.
3. Berman, P.W., Groopman, J.E., Gregory, T., Clapham, P.R., Weiss, R.A., Ferriani, R. Riddle, L., Shimasaki, C., Lucas, C., Lasky, L.A. & Eichberg, J.W. (1988) Proc. Natl. Acad. Sci. U.S.A. 85 5200-5204.
4. Arthur, L.O., Bess, J.W., Waters, D.J., Pyle, S.W., Kelliher, J.C., Nara, PL., Krohn, K.,Robey, W.G., Langlois, A.J., Gallo, R.C. & Fischinger, P.J. (1989) J. Virol. 63,5046-5053.
5. Girard, M., Kieny, M.P., Gluckman, J.C., Barre-Sinoussi, F., Montagnier, L. & Fultz, P. (1990) in Vaccines for Sexually Transmitted Diseases eds. Meheus, A. & Spier, R. (Butterworth Co., Ltd., London), pp. 227-237.
6. Hu, S.L. Fultz, P.N., McClure, H.M., Eichberg, J.W., Thomas, E.K., Zarling, J., Singhal, M.C., Kosowski, S.G., Swenson, R.B., Anderson, D., C. & Todaro, G. (1987) Nature (London) 328, 721-723.
7. Berman, P.W., Gregory, T.J., Riddle, L., Nakamura, G.R., Champe, M..A., Porter, J.P., Wurm, F.M., Hershberg, R.D., Cobb, E.K. & Eichberg, J. W. (1990) Nature (London) 345, 622-625.
8. Desrosiers, R.C., Wyand, M.S., Kodama, T, Ringler, D.J., Arthur, L.O., Sehgal, P.K., Letvin, N.L., King, N.W. & Daniel, M.D. (1989) Proc. Natl. Acad. Sci. U.S.A. 86 86, 6353-6357.
9. Murphey-Corb, M., Martin, L.M., Davison-Fairburn, B., Montelaro, R.C., Miller, M., West, M., Ohkawa, S., Baskin, G.B., Zhang, J.Y, Putney, S., D. Allison, A.C. & Eppstein, D.A. (1989) Science 246, 1293-1297.
10. Emini, E.A., Nara, PL., Schleif, W.A., Lewis, J.A., Davide, J.P, Lee, D.R., Kessler, J., Conley, S., Matsushita, S., Putney, S.D., Gerety, R.J. & Eichberg, J.W. (1990) J. Virol. 64, 3674-3678.
11. Moor-Jankowski, J. & Mahoney, C.J. (1989) J. Med. Primatol. 18, 1-26.
12. Kieny, M.P., Lathe R., Riviere, Y, Dott, K., Schmitt, D., Girard, M., Montagnier, L. & Lecocq. J.R (1988) Prot. Engineering 2, 219-226.
13. Schmidt, D., Dezutter-Dambuyant, C., Hanau, D., Schmitt, D.A., Kolbe, H.V.J., Kieny, M.R, Cazenave, J.R & Thivolet, J. (1989) Comptes Rendus Acad. Sci. Paris, 308(III), 269-275.
14. Guy, B., Riviere, Y, Dott, K. Regnault, A. & Kieny, M.P. (1990) Virology 176, 413-425.
15. Kolbe, H.V, Jaeger, F., Lepage, P., Roitsch, C., Lacaud, G., Kieny, M.P., Sabatie, J., Brown, S.W. & Lecocq, J.P. (1989) J. Chromatography 476, 99-112.
16. Allison, A.C. & Byars, N.E. (1986) J. Immunol. Methods 95, 157-168.
17. Putney, S.D., Matthews, T.J., Robey, W.G., Lynn, D.L., Robert-Guroff, M., Mueller, W.T., Langlois, A.L., Ghrayeb, J., Petteway, S.R., Weinhold, K.J., Fischinger, P.J., Wong-Staal, F., Gallo, R.C. & Bolognesi, D.R (1986) Science 234, 1392-1395.
18. Rusche, J.R., Kavaherian, K., McDanal, C., Petro, J., Lynn, D.L., Grimaila, R., Langlois, A., Gallo, R.C., Arthur, L.O., Fischinger, P.J., Bolognesi, D.R, Putney, S.D. & Matthews, T.J. (1988) Proc. Natl. Acad Sci. U.S.A. 85, 3198-3202.
19. LaRosa, G.J., Davide, J.R, Weinhold, K., Waterbury, J.A., Profy, A.T., Lewis, J.A., Langlois, A.J., A.J., Dressman, G.R. Boswell, R.N., Shadduck, P., Holley, L.H., Karplus, M., Bolognesi, D.R, Matthews, T.J. Emini, E.A. & Putney, S.D. (1990) Science 249 932-935.
20. Nara, P.L., Hatch, W.C., Dunlop, N.M., Robey, W.G., Arthur, L.O., Gonda, M.A. & Fischinger, P.J. (1987) AIDS Res. Human Retroviruses 3, 283-302.
21. Fultz, PN., McClure, H.M., Swenson, R.B., McGrath, C.R., Brodie, A., Getchell, J.R, Jensen, FC., Anderson, D.C., Broderson, J.R. & Francis, D.P. (1986) J. Virol., 58, 116-124.
22. Laure, F., Rouzioux, C., Veber, F., Jacomet, C., Courgnaud, V., Blanche, S., Burgard, M., Griscelli, C. & Brechot, C. (1988) Lancet 2, 538-541.
23. Mullis, K.B. & Faloona, F.A. (1987) Methods Enzymol. 155, 335-350.
24. Kwok, S. & Kellogg, D.E. (1990) in PCR Protocols: A Guide to Methods and Applications: eds. Innis, M.A., Gelfand, D.H., Sninsky, J.J. & White T.J. (Academic Press, Inc., San Diego, CA) pp. 337-347.
25. Zagury, D., Bernard, J., Cheynier, R., Desportes, I., Leonard, R., Fouchard, M., Reveil, B., Ittele, F.D., Lurhama, Z., Mbayo, K., Wane, J., Salaun, J.J., Goussard, B., Dechazal, L., Burny, A., Nara, P. & Gallo, R.C. (1988) Nature (London) 322, 728-731.
26. Nara, PL., (1989) in Vaccines 89, eds. Lerner, R.A., Ginsberg, H., Chanock, R.M. & Brown, F. (Cold Spring Harbor Laboratory, Cold Spring Harbor, NY) pp. 137-144.
27. Myers, G. (1990) in Human Retroviruses and AIDS, eds. Myers, G., Josephs, S.F., Wong-Staal, F., Rabson, A.B., Smith, T.F. & Berzofsky, J.A. (Los Alamos National Laboratory, Los Alamos, NM).
28. Scharf, S.J., Horn, G.T & Erlich, H.A. (1986) Science 233, 1076-1078.
29. Walker, C.M., Moody, D.J., Stites, D.P. & Levy, J.A. (1986) Science 234, 1563-1566.
30. Tsubota, H., Lord, C.I., Watkins, D.I., Morimoto, C. & Letvin, N.L. (1989) J. Exp. Med. 169, 1421-1434.
31. Ranki, A., Valle, S.L., Krohn, M., Antonen, J., Allain, J.P., Leuther, M., Franchini, G. & Krohn, K. (1987) Lancet 2, 589-593.
32. Jehuda-Cohen, T, Slade, B.A., Powell, J.D., Villinger, F., De, B., Folks, TM., McClure, H.M., Sell, K.W. & Ahmed-Ansari, A. (1990) Proc. Natl. Acad. Sci. U.S.A. 87, 3972-3976.
33. Bahraoui, E., Yagello, M., Billaud, J.N., Sabatier, J.M., Guy, B., Muchmore, E., Girard, M. & Gluckman, J.C. (1990) AIDS Res. Human Retroviruses 6, 1087-1088.
34. Van Eendenburg, J.R, Yagello, M., Girard, M., Kieny, M.R, Lecocq, J.R, Muchmore, E., Fultz, P.N., Riviere, Y, Montagnier, L. & Gluckman, J.C. (1989) AIDS Res. Human Retroviruses 5, 41-50.

## Claims

1. Monoclonal or polyclonal antibodies having neutralizing properties regarding the infection by a determined virus, obtained after administration to a host, in an amount sufficient to induce neutralizing antibodies, of a composition comprising as a combined preparation for simultaneous, separate or sequential administration:
(A) at least one envelope glycoprotein of said virus or a fragment of at least 50 aminoacids of the glycoprotein and,
(B) at least one peptide derived from the aminoacid sequence of the envelope glycoprotein, and wherein the peptide comprises at least one virus-neutralization epitope.

2. Monoclonal or polyclonal antibodies according to claim 1 wherein the administered composition comprises as a combined preparation for simultaneous separate or sequential use:
(A) at least one envelope glycoprotein of the virus or a fragment of the glycoprotein having its immugenic properties in an amount sufficient for priming the induction of neutralizing antibodies in the host to which the envelope glycoprotein is administered; and
(B) at least one peptide derived from the amino acid sequence of said envelope glycoprotein, wherein the peptide comprises at least one virus-neutralization epitope of said glycoprotein and said composition contains said peptide in an amount sufficient to enhance the induction of persistent neutralizing antibodies in the host.

3. Monoclonal antibodies or polyclonal antibodies according to claim 1 or 2, wherein the virus is selected from the group consisting of HIV, SIV, HTLV-1, HTLV-2, FIV, FeLV

4. Monoclonal antibodies or polyclonal antibodies according to anyone of claims 1 to 3, wherein the envelope glycoprotein is the gp160 of HIV, or gp120 of HIV, or fragments of these glycoproteins.

5. Monoclonal antibodies or polyclonal antibodies according to anyone of claims 1 to 4, wherein said at least one peptide comprises a mixture of peptides of glycoprotein of HIV.

6. Monoclonal antibodies or polyclonal antibodies according to anyone of claims 1 to 5, wherein said at least one peptide is bound to a carrier molecule for example an alphatic sequence.

7. Monoclonal antibodies or polyclonal antibodies according to anyone of claims 1 to 6, wherein said at least one envelope glycoprotein comprises a mixture of HIV-1 and HIV-2 glycoproteins, and said at least one peptide comprises a mixture containing at least one peptide having an HIV-2 neutralization epitope.

8. Monoclonal antibodies or polyclonal antibodies according to anyone of claims 1 to 7, wherein said at least one glycoprotein is a mixture of glycoproteins gp160 from different HIV isolates (serotypes) and said at least one peptide is a mixture of the corresponding neutralization epitopes.

9. Monoclonal antibodies or polyclonal antibodies according to anyone of claims 1 to 7, wherein said at least one envelope glycoprotein is a mixture of glycoproteins gp120 from different HIV isolates (serotypes) and said at least one peptide is a mixture of the corresponding neutralization epitopes.

10. Monoclonal antibodies or polyclonal antibodies according to anyone of claims 1 to 9, wherein the peptide is selected from the group consisting of env, gag, and especially p18gag, nef, vif, pol or GPG or GLG antigens, and mixtures of said antigens, particularly p27nef and p23vif.

11. Monoclonal antibodies or polyclonal antibodies according to anyone of claims 1 to 10, wherein the envelope glycoprotein of a determined virus is combined with at least one of the antigens selected among gag, pol, nef, or vif and particularly with a mixture of p27nef and p23vif.

12. Monoclonal antibodies or polyclonal antibodies according to anyone of claims 1 to 10 peptide is at least one peptide selected from the group consisting of:
C-TRPNNNTRKR IRIQRGPGRA FVTIGK-IGN M-RQAH-C
C-TRPNNNTRKS IRIQRGPGRA FVTIGK-IGN M-RQAH-C
C-TRPNNNTRKK IRIQRGPGRA FVTIGK-IGN M-RQAH-C
C-TRPNNNTRGS IRIQRGPGRA FVTIGK-IGN M-RQAH-C
C-TRPNNNTRKS IYI--GPGRA FHTTGRIIGD -IRKAH-C
C-TRPYNNVRRS LSI--GPGRA FRTRE-IIGI -IRQAH-C
C-TRPGNNTRRG IHF--GPGQA LYTTGIV-GD -IRRAY-C
C-ARPYQNTRQR TPI--GLGQS LYTTRSR-SI -IGQAH-C
C-TRPNNNTRKS ITK--GPGRV IYATGQIIGD -IRKAH-C
C-TRPNNNTRKR ITM--GPGRV YYTTGQIIGD -IRRAH-C
C-TRPGSDKRQS TPI--GLGQA LYTTRGRTKI - IGQAH-C
C-TRPGSDKKIR QSIRIGPGKV FYAKGG---I -TGQAH-C
C-TRPNNNTKKG IAI--GPGRT LYAREKIIGD -IRQAH-C
C-TRPNNHTRKR VTL--GPGRV WYTTGEILGN -IRQAH-C
C-TRPGNNTRRG SHF--GPGQA LYTTGIVGDI -RRAYC
C-TRPDNKITSRQ-TPI-GLGQA LYTTRIKGDI -RQAY-C
C-TRPNNNVRRR-HIHI-GPGRA FYTGEIRNI -RQAH-C
C-TRPYKNTRQS-TPI--GLGQA LYTTRTKSI -GQAH-C
C-TRPNNNTTRS-IHI--GPGRA FYATGDIIGTIRQAH-C
C-TRPNYNKRKR-IHI--GPGRA FYTTKNIIGDIRQAH-C

13. Monoclonal antibodies or polyclonal antibodies according to anyone of claims 1 to 10, wherein the peptide comprises the following amino acid sequence:
YNTRKSIRIQRGPGRAFVIGKIGN

14. Monoclonal antibodies or polyclonal antibodies according to anyone of claims 1 to 10, at least one peptide is comprised of the major neutralization epitope (loop V3) of at least one HIV-1 isolate.

15. Monoclonal antibodies or polyclonal antibodies according to anyone of claims 1 to 14, wherein the composition contains an adjuvant in an amount sufficient to prime and/or to enhance the immunogenicity of the envelope glycoprotein.

16. Monoclonal antibodies or polyclonal antibodies according to claim 15, wherein the adjuvant is muramyl dipeptide Syntex adjuvant SAF-1 or incomplete Freund's adjuvant.

17. Monoclonal antibodies or polyclonal antibodies according to anyone of claims 1 to 16, **characterized in that** it is orally, parenterally, or intradermally administered.

18. Monoclonal antibodies or polyclonal antibodies according to anyone of claims 1 to 17, having a neutralization activity determined by inhibition of syncytia formation in CEM-SS cells, above 64.

19. A mixture of monoclonal and/or polyclonal antibodies according to anyone of claims 1 to 18.

20. Use of monoclonal antibodies or polyclonal antibodies according to anyone of claims 1 to 18 as serotherapeutic drug.

21. Monoclonal or polyclonal antibodies according to anyone of claims 1 to 19, having a neutralizing titer higher than 1/64 as measured to obtain 90% inhibition of syncytia in CEM-SS cells.
